# EUROPEAN PATENT SPECIFICATION

(11) **EP 4 499 160 B1**
(45) Date of publication and mention of the grant of the patent: **24.09.2025**
(21) Application number: 23716252.4
(22) Date of filing: 29.03.2023
(51) Int. Cl.: A61L 27/20, A61L 27/38, A61L 27/52, A61L 27/58, A61F 2/00

(54) **USE OF POLYMER-BASED MICROCARRIERS IN THE PRODUCTION OF TISSUE SCAFFOLDS WITH COMPLEX GEOMETRY**
VERWENDUNG VON MIKROTRÄGERN AUF POLYMERBASIS BEI DER PRODUKTION VON GEWEBEGERÜSTEN MIT KOMPLEXER GEOMETRIE
UTILISATION DE MICROPORTEURS À BASE DE POLYMÈRES DANS LA PRODUCTION D'ÉCHAFAUDAGES DE TISSUS À GÉOMÉTRIE COMPLEXE

(30) Priority: 30.03.2022 EP 22165646
(43) Date of publication of application: 05.02.2025
(73) Proprietor: NORDOVO BIOSCIENCES AS, 0585 Oslo (NO)
(72) Inventor: SCHWEIKLE, Manuel, 0585 Oslo (NO); RAMBØL, Mia Hauge, 0585 Oslo (NO); HÅTI, Armend Gazmeno, 0585 Oslo (NO)
(74) Representative: Acapo Onsagers AS
(86) International application number: PCT/EP2023/058211
(87) International publication number: WO 2023/187018

(56) References cited:
- WO-A1-2017/223529
- WO-A1-2018/213375
- RUBIN J P ET AL: "Collageneous microbeads as a scaffold for tissue engineering with adipose-derived stem cells", PLASTIC AND RECONSTRUCTIVE SURGERY, WOLTERS KLUWER HEALTH, US, vol. 120, no. 2, 1 August 2007 (2007-08-01), pages 414 - 424, XP008129233, ISSN: 0032-1052, DOI: 10.1097/01.PRS.0000267699.99369.A8

## Description

### FIELD THE INVENTION

The present invention relates to regenerative medicine in general, in particular tissue engineering. The present invention provides improved manufacturing methodologies which enables production of complex three-dimensional tissue scaffolds. The scaffolds are essentially non-immunogenic when implanted into a subject, mimic native extracellular matrix and may be repopulated and modulated by host cells thereby becoming living tissues.

### BACKGROUND OF THE INVENTION

Transplantation from human donors is currently the primary source for replacing damaged or dysfunctional organs and tissues in the clinic. Although transplantation has become a successful practice worldwide the approach holds significant limitations by severe shortage of donors, complexity of the procedure and potential risk for transmission of infectious agents. As such the use of animal tissues or synthetic materials to produce grafts have emerged as possible alternatives to human tissues. However, the disadvantages of such procedures are many and among the most important are the immunological barriers. Hence, there is a need for innovative approaches in regenerative medicine to increase the number of patients that may receive clinical implantations and with a higher success rate.

Tissue engineering is a continuously growing multidisciplinary field applying the principles of engineering and life science to produce functional replacement tissues for clinical use. The field seeks to develop biological substitutes to restore, maintain or improve the biological function of a tissue or whole organ. Moreover, such tissue engineered constructs may also serve as in vivo systems for delivery of cell-secreted molecules or treatment agents, or as in vitro models of tissue function to test various treatment effects.

In general, tissue engineering comprises methods of culturing cells on a supportive matrix (scaffold) to form new tissues of intended shape and function. The supportive matrix should thus be processable to form scaffolds of a desired shape for the tissue of interest, and the cells can be obtained from the recipient to decrease the likelihood of immune system rejection or other biocompatible sources such as established human cell lines. However, to date the successful clinical implantation of such engineered tissues is low, and innovative approaches are needed to improve methodology and increase the translational value of the products.

The tissue scaffold that serves as an intermediate in the production of a new functional tissue may present a risk of immunologic and/or inflammatory reactions when implanted. Hence, with the aim to produce more biocompatible scaffolds, methods are currently investigated to develop human decellularized extracellular matrix tissues as supportive matrices for tissue culture. Extracellular matrix proteins from humans are expected to be essentially non-immunogenic when implanted into a human and mimic natural extracellular matrix compositions to achieve necessary functional tissue properties. Further, the human decellularized extracellular matrix tissues can be repopulated and modulated by host cells thereby becoming living tissues. This makes human extracellular matrix proteins preferable to proteins derived from animal sources and to synthetic degradable or non-degradable polymers as tissue scaffolds.

To date, such tissue engineered decellularized extracellular matrix scaffolds are produced by culturing human cells on tubular biodegradable polyglycolic acid (PGA) substrates, that degrades as the cells produce extracellular matrix proteins (WO2012/094611). The tubular biodegradable PGA constructs are produced by way of wrapping a biodegradable PGA sheet around a mandrel (gas permeable, silicone tube) such that opposite edges of the PGA sheet meet at an interface. At the interface PGA fibers are pulled from each opposing edge of the sheet and entangled to form a seam creating a tubular construct of uniform density. As the extracellular matrix proteins have been secreted on the tubular PGA construct, the construct is decellularized such that the construct is substantially acellular, leaving an extracellular matrix construct.

There are several shortcomings of this approach. First and foremost, the procedure using tubular PGA scaffolds is primarily tailored to forming grafts of rather simple geometry, such as vascular grafts, limiting the potential use of the technique for replacement of other more complex dysfunctional tissues or organs. Secondly, the methodology requires complex and time-consuming manual work going from a PGA sheet to a vascular graft. Complex procedures often make it difficult to avoid variation between products, and the seam connecting the sheets will represent a weak link in the construct. Thirdly, the procedure depends on treating the tubular PGA construct to increase the rate of degradation and on the cultured cells to degrade and thus remove the supportive PGA matrix. The residual percentage of PGA matrix might present immunogenic and/or inflammatory reactions when implanted. Fourthly, using a macroporous PGA membrane limits how thick the final decellularized extracellular matrix construct can be. This again limits the potential use in replacement of various dysfunctional tissues and organs. Consequently, there is a need in the art to develop techniques that improve the tissue scaffolds for use in tissue engineering.

WO0214480 address that such decellularized tissue engineered constructs, as produced in WO2012/09461, can be configured to assume any desired three-dimensional shape, however, fails to disclose how such three-dimensional shapes can be made from the original biodegradable PGA sheet other than the tubular shape. Hence, there is a need in the art to enable production of complex three-dimensional tissue scaffolds for use in tissue engineering.

WO2020/045162 discloses a drug delivery system (DDS) in the form of a sustained release composition comprising drugs encapsulated in alginate microbeads with a diameter of about 80 µm. In order to investigate how the DDS affects cell viability, the microbeads are mixed with cells and thereafter subjected to alginate lyase treatment to dissolve the microbeads. However, alginate without any grafted cell adhesion ligands does not support attachment of cells.

WO2017/223529 discloses methods of manufacturing tissue matrix scaffolds (TMS) which may be used as three-dimensional cell culture environments to culture cells or tissues. The TMS hydrogel is produced by extracting extracellular matrix (ECM) from homogenized animal tissue and concentrating the ECM extract. The concentrated ECM extract may be dehydrated to obtain porous TMS. Similar methodology is also described in WO2018/213375.

Plastic and reconstructive surgery, Wolters Kluwer Health, US, vol. 120, no. 2, 1 August 2007, pages 414-424, XP008129233 suggests that porous collagenous beads could be useful as injectable cell delivery vehicles for adipose-derived stem cells and that future in-vivo studies will examine the potential of "adipose derived stem cells attached to porous collagenous beads" to form adipose tissue.

### Summary of the invention

The present inventors have solved the above needs by providing improved manufacturing methodologies which enables production of complex three-dimensional tissue scaffolds. These scaffolds are essentially non-immunogenic when implanted into a human, mimic native extracellular matrix and may be repopulated and modulated by host cells thereby becoming living tissues.

A **first aspect** of the present invention relates to a method of manufacturing a tissue scaffold, the method comprising the following steps:
a) providing a first group of cells and biocompatible hydrogel beads;
b) bringing the first group of cells together with the biocompatible hydrogel beads to obtain a first cell-bead mixture;
c) culturing the first cell-bead mixture to produce extracellular matrix thereby obtaining a first tissue scaffold;
d) optionally, subjecting the first tissue scaffold to mechanical conditioning thereby obtaining a mechanically conditioned first tissue scaffold;
wherein
- the first group of cells are anchorage dependent cells that can produce and secrete extracellular matrix;
- the biocompatible hydrogel beads support attachment of the first group of cells; and
- the first group of cells and the biocompatible hydrogel beads of step b) are brought together in a mold to obtain the first cell-bead mixture; or
   the first cell-bead mixture of step b) is incubated to allow formation of bead-associated cells and then transferred into a mold; or
   the first group of cells and the biocompatible hydrogel beads of step b) are incubated to allow formation of bead-associated cells, the bead-associated cells then being transferred into a mold to obtain the first cell-bead mixture.

In one embodiment, the first group of cells and the biocompatible hydrogel beads of step b) are brought together in a mold to obtain the first cell-bead mixture; or the first cell-bead mixture of step b) is incubated to allow formation of bead-associated cells and then transferred into a mold.

In another embodiment, the first group of cells and the biocompatible hydrogel beads of step b) are brought together in a mold to obtain the first cell-bead mixture; or the first group of cells and the biocompatible hydrogel beads of step b) are incubated to allow formation of bead-associated cells, the bead-associated cells then being transferred into a mold to obtain the first cell-bead mixture.

In one embodiment, the first cell-bead mixture in step c) is cultured at least until the first tissue scaffold can be removed from the mold and still maintain its shape, i.e. that the first tissue scaffold is self-supporting. Example 2 provides methodology which may be used to decide when a tissue scaffold is self-supporting.

### Further step(s)

In one embodiment, step d) is mandatory.

In one embodiment, the first tissue scaffold or the mechanically conditioned first tissue scaffold is subjected to decellularization thereby obtaining a **second tissue scaffold.**

In one embodiment, the second tissue scaffold is subjected to mechanical conditioning thereby obtaining a mechanically conditioned second tissue scaffold.

In one embodiment, the second tissue scaffold or the mechanically conditioned second tissue scaffold is incubated in the presence of a second group of cells, thereby obtaining a second tissue scaffold repopulated with the second group of cells.

In one embodiment,
- the first tissue scaffold or the mechanically conditioned first tissue scaffold is subjected to means for dissolving or degrading the biocompatible hydrogel beads, thereby obtaining a **third tissue scaffold;** or
- the biocompatible hydrogel beads are self-degradable or self-dissolvable; and the first tissue scaffold or the mechanically conditioned first tissue scaffold is cultured until the biocompatible hydrogel beads are self-dissolved or self-degraded, thereby obtaining a **third tissue scaffold.**

In one embodiment, the third tissue scaffold is subjected to mechanical conditioning thereby obtaining a mechanically conditioned third tissue scaffold.

In one embodiment, the third tissue scaffold or the mechanically conditioned third tissue scaffold is incubated in the presence of a second group of cells, thereby obtaining a third tissue scaffold repopulated with the second group of cells.

In one embodiment, the third tissue scaffold or the mechanically conditioned third tissue scaffold is subjected to decellularization thereby obtaining a **fourth tissue scaffold.**

In one embodiment, the fourth tissue scaffold is subjected to mechanical conditioning thereby obtaining a mechanically conditioned fourth tissue scaffold.

In one embodiment, the fourth tissue scaffold or the mechanically conditioned fourth tissue scaffold is incubated in the presence of a second group of cells, thereby obtaining a fourth tissue scaffold repopulated with the second group of cells.

In a first preferred embodiment according to the first aspect of the present invention, the method further comprises the following step(s):
e) subjecting the first tissue scaffold or the mechanically conditioned first tissue scaffold to means for dissolving or degrading the biocompatible hydrogel beads, thereby obtaining a **third tissue scaffold;**
f) optionally, subjecting the third tissue scaffold to mechanical conditioning thereby obtaining a mechanically conditioned third tissue scaffold;
g) subjecting the third tissue scaffold or the mechanically conditioned third tissue scaffold to decellularization thereby obtaining a **fourth tissue scaffold;**
h) optionally, subjecting the fourth tissue scaffold to mechanical conditioning thereby obtaining a mechanically conditioned fourth tissue scaffold; and
i) optionally, incubating the fourth tissue scaffold or the mechanically conditioned fourth tissue scaffold in the presence of a second group of cells, thereby obtaining a fourth tissue scaffold repopulated with the second group of cells.

In a preferred embodiment, at least one of the following steps is mandatory: step f), step h) and step i). In a more preferred embodiment, at least two of the following steps are mandatory: step f), step h) and step i). In an even more preferred embodiment step f), step h) and step i) are mandatory. In the most preferred embodiment, step f) and i) are mandatory.

In one embodiment, step f) is mandatory.

In one embodiment, step h) is mandatory.

In one embodiment, step i) is mandatory.

In one embodiment, the third tissue scaffold comprises extracellular matrix embedding the first group of cells and hollow bodies previously occupied by the biocompatible hydrogel beads.

In a preferred embodiment, the third tissue scaffold is cultured to i) produce extracellular matrix within hollow bodies previously occupied by the biocompatible hydrogel beads; and/or ii) facilitate migration of the first group of cells into the hollow bodies; iii) and/or allow contractile forces of the first group of cells to close the hollow bodies. It is preferred that the hollow bodies are filled with extracellular matrix and/or the first group of cells. It is particularly preferred that the hollow bodies are filled with extracellular matrix and/or the first group of cells prior to step f) and/or step g).

In a second preferred embodiment according to the first aspect of the present invention, the method further comprises the following step(s):
e) the biocompatible hydrogel beads are self-degradable or self-dissolvable; and the first tissue scaffold or the mechanically conditioned first tissue scaffold is cultured until the biocompatible hydrogel beads are self-dissolved or self-degraded, thereby obtaining a **third tissue scaffold;**
f) optionally, subjecting the third tissue scaffold to mechanical conditioning thereby obtaining a mechanically conditioned third tissue scaffold;
g) subjecting the third tissue scaffold or the mechanically conditioned third tissue scaffold to decellularization thereby obtaining a **fourth tissue scaffold;**
h) optionally, subjecting the fourth tissue scaffold to mechanical conditioning thereby obtaining a mechanically conditioned fourth tissue scaffold; and
i) optionally, incubating the fourth tissue scaffold or the mechanically conditioned fourth tissue scaffold in the presence of a second group of cells, thereby obtaining a fourth tissue scaffold repopulated with the second group of cells.

In a preferred embodiment, at least one of the following steps is mandatory: step f), step h) and step i). In a more preferred embodiment, at least two of the following steps are mandatory: step f), step h) and step i). In an even more preferred embodiment step f), step h) and step i) are mandatory. In the most preferred embodiment, step f) and i) are mandatory.

In one embodiment, step f) is mandatory.

In one embodiment, step h) is mandatory.

In one embodiment, step i) is mandatory.

In one embodiment, the third tissue scaffold comprises extracellular matrix embedding the first group of cells and hollow bodies previously occupied by the biocompatible hydrogel beads.

In a preferred embodiment, the third tissue scaffold is cultured to i) produce extracellular matrix within hollow bodies previously occupied by the biocompatible hydrogel beads; and/or ii) facilitate migration of the first group of cells into the hollow bodies; iii) and/or allow contractile forces of the first group of cells to close the hollow bodies. It is preferred that the hollow bodies are filled with extracellular matrix and/or the first group of cells. It is particularly preferred that the hollow bodies are filled with extracellular matrix and/or the first group of cells prior to step f) and/or step g).

### Cells

In one embodiment the first group of cells is selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells and in particular adipose tissue derived mesenchymal stem cells or any combination thereof.

The second group of cells may be the same or different from the first group of cells.

In one embodiment, the second group of cells is selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells and in particular adipose tissue derived mesenchymal stem cells or any combination thereof.

In one embodiment, the second group of cells is different from the first group of cells and is selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells and in particular adipose tissue derived mesenchymal stem cells or any combination thereof.

It is preferred that the second group of cells are anchorage dependent cells.

In one embodiment according to the present invention, the second group of cells is progenitor cells or adult stem cells (ASCs). These cells are preferably allowed to differentiate within the tissue scaffold in question to develop into the desired tissue or organ.

### Biocompatible hydrogel beads

According to the first aspect, the first group of cells are brought together with the biocompatible hydrogel beads to obtain a first cell-bead mixture, cf. step (b) of the above method.

In one embodiment according to the present invention, the biocompatible hydrogel beads are degradable or dissolvable.

In one embodiment according to the present invention, the biocompatible hydrogel beads are self-degradable or self-dissolvable.

In one embodiment according to the present invention, the biocompatible hydrogel beads have one or more cell-adhesion ligand(s) on their outer surface. The one or more cell-adhesion ligand(s) may e.g. be i) a peptide comprising a cell adhesive amino acid sequence, such as RGD, YIGSR, GFOGER or PHSRN; or ii) a charged polymer, such as poly-L-Lys. The one or more cell-adhesion ligand(s) preferably being incorporated into the biocompatible hydrogel beads via covalent tethering, electrostatic interactions, and/or interpenetrating networks.

In one embodiment, the one or more cell-adhesion ligand(s) is a peptide comprising the amino acid sequence RGD, such as a peptide comprising the amino acid sequence GRGDSP.

In one embodiment, the biocompatible hydrogel beads comprise alginate, fibrin, gelatin, agarose, modified or unmodified poly-ethylene glycol, carrageenan, pectin or any combination thereof.

In one embodiment, the biocompatible hydrogel beads are made from alginate, fibrin, gelatin, agarose, modified or unmodified poly-ethylene glycol, carrageenan, pectin or any combination thereof.

In a preferred embodiment, the biocompatible hydrogel beads are biocompatible alginate beads. The biocompatible alginate beads have preferably been produced by competitive ligand exchange crosslinking as described in e.g. example 1 of WO2017153947.

In another preferred embodiment, the biocompatible hydrogel beads are biocompatible hydrogel beads comprising alginate. The biocompatible hydrogel beads comprising alginate have preferably been produced by competitive ligand exchange crosslinking as described in e.g. example 3 of WO2017153947.

In one embodiment, the biocompatible hydrogel beads are biocompatible alginate beads and the alginate is RGD-coupled alginate or GRGDSP-coupled alginate.

In one embodiment, the biocompatible hydrogel beads are biocompatible alginate beads and the means for dissolving or degrading the biocompatible hydrogel alginate beads is selected from the group consisting of alginate lyase treatment; treatment with a divalent cation chelating agent; and any combination thereof.

In one embodiment, the biocompatible hydrogel beads are biocompatible hydrogel microbeads, preferably biocompatible hydrogel microbeads of an average size less than 500 µm in their largest dimension; more preferably of an average size in the range 10 to 200 µm, such as 10 to 150 µm or 30 to 150 µm, in their largest dimension; even more preferably of an average size in the range 30 to 90 µm, such as 30 to 80 µm, in their largest dimension; and most preferably of an average size in the range 50 to 80 µm in their largest dimension. The size of the beads is measured at their swollen equilibrium size in a liquid environment of physiological ion concentration, such as cell culture medium; see section 1.2 of example 1.

In one embodiment, at least 60 %, such as at least 65 %, at least 70 %, at least 75 %, at least 80 %, at least 85 %, at least 90 %, at least 95 % or at least 99 %, of the biocompatible hydrogel beads have a size in the range 10 to 200 µm in their largest dimension, such as a size in the range 10 to 150 µm in their largest dimension, a size in the range 30 to 150 µm in their largest dimension or a size in the range 30 to 90 µm in their largest dimension. Thus, the person skilled in the art will understand that if there are 100 000 biocompatible hydrogel beads and 60 % of the biocompatible hydrogel beads have a size in the range 10 to 200 µm in their largest dimension there are in fact 60 000 biocompatible hydrogel beads which have a size in the range 10 to 200 µm in their largest dimension.

### Mold

In one embodiment the mold is made of a bioinert material.

In one embodiment, the mold has one or more of the following properties:
- allow control of geometry;
- prevent cell attachment or cell infiltration; and
- allow exchange of nutrients and gases between exterior and interior of the mold.

In one embodiment, the mold is designed to prevent cell attachment or infiltration.

In one embodiment, the mold allows exchange of nutrients and/or gases between exterior and interior of the mold.

In one embodiment, the mold is completely or partly submerged in cell culture media.

In a more preferred embodiment, the mold is designed to prevent cell attachment or infiltration; allows exchange of nutrients and gases between exterior and interior of the mold; and is completely or partly submerged in cell culture media.

In one embodiment, the mold and the first tissue scaffold has substantially the same shape as the first tissue scaffold.

In one embodiment, the mold has the shape of an annular well.

In one embodiment, the mold comprises a container and at least one element which is located inside the container. The container and the at least one element may have any geometrical shape. The space inside the container, which is not occupied by the at least one element, is suitable for culturing the first cell-bead mixture to produce extracellular matrix thereby obtaining a first tissue scaffold. The at least one element may be included inside the container before the first group of cells and the biocompatible hydrogel beads are brought together in the mold; or the first group of cells and the biocompatible hydrogel beads are brought together inside the container and thereafter the at least one element is included inside the container. In case of the latter, the at least one element should be included inside the container before culturing to produce extracellular matrix (step c). The at least one element may be a prefabricated element which is attached to the container or may be produced by 3D printing on the surface of the container. The person skilled in the art is familiar with suitable 3D-printing techniques, including suspended layer additive manufacturing (Adv. Funct. Mater. 2019, 29, 1904845; Lee et al., Science 365, 482-487, 2019).

A **second aspect** of the present invention relates to a first tissue scaffold comprising
- a first group of cells;
- biocompatible hydrogel beads supporting attachment of the first group of cells; and
- extracellular matrix produced by the first group of cells;
wherein
- the first group of cells are anchorage dependent cells that can produce and secrete extracellular matrix;
- the first group of cells are attached to an outer surface of the biocompatible hydrogel beads; and
- the first group of cells and the biocompatible hydrogel beads are embedded in the extracellular matrix.

In one embodiment according to the second aspect of the present invention, neighboring biocompatible hydrogel beads are connected to one another by a network of the first group of cells and the extracellular matrix.

In one embodiment according to the second aspect of the present invention, the first tissue scaffold has a tubular shape, such as a shape of a circular tube.

In one embodiment according to the second aspect of the present invention, the first tissue scaffold is obtained by the method according to the first aspect of the present invention.

In one embodiment according to the second aspect of the present invention, the first tissue scaffold has been subject to mechanical conditioning thereby obtaining a mechanically conditioned first tissue scaffold.

In one embodiment according to the second aspect of the present invention, the first tissue scaffold or the mechanically conditioned first tissue scaffold has been repopulated by a second group of cells.

In one embodiment according to the second aspect of the present invention, the first tissue scaffold or the mechanically conditioned first tissue scaffold may be used for reproducing a functional organ by repopulating the scaffold with a second group of cells. The second group of cells may be allowed to differentiate within the first tissue scaffold to develop into the desired tissue.

In one embodiment according to the second aspect of the present invention, the first tissue scaffold or the mechanically conditioned first tissue scaffold is suitable for being transplanted into a subject, such as a human subject.

### Cells

In one embodiment according to the second aspect of the present invention, the first group of cells is anchorage dependent cells, more preferably human anchorage dependent cells.

In one embodiment according to the second aspect of the present invention, the first group of cells are selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells and in particular adipose tissue derived mesenchymal stem cells or any combination thereof.

The second group of cells may be the same or different from the first group of cells.

In one embodiment, the second group of cells is selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells and in particular adipose tissue derived mesenchymal stem cells or any combination thereof.

In one embodiment, the second group of cells is different from the first group of cells and is selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells and in particular adipose tissue derived mesenchymal stem cells or any combination thereof.

It is preferred that the second group of cells are anchorage dependent cells.

In one embodiment according to the present invention, the second group of cells is progenitor cells or adult stem cells (ASCs). These cells are preferably allowed to differentiate within the tissue scaffold in question to develop into the desired tissue or organ.

### Biocompatible hydrogel beads

In one embodiment according to the second aspect of the present invention, the biocompatible hydrogel beads have one or more cell-adhesion ligand(s) on their outer surface; and the first group of cells are attached to an outer surface of the biocompatible hydrogel beads through interaction with the one or more cell-adhesion ligand(s).

In one embodiment according to the second aspect of the present invention, the biocompatible hydrogel beads are degradable or dissolvable.

In one embodiment according to the second aspect of the present invention, the biocompatible hydrogel beads are self-degradable or self-dissolvable.

In one embodiment according to the second aspect of the present invention, the biocompatible hydrogel beads have one or more cell-adhesion ligand(s) on their outer surface. The one or more cell-adhesion ligand(s) may e.g. be i) a peptide comprising a cell adhesive amino acid sequence, such as RGD, YIGSR, GFOGER or PHSRN; or ii) a charged polymer, such as poly-L-Lys. The one or more cell-adhesion ligand(s) preferably being incorporated into the biocompatible hydrogel beads via covalent tethering, electrostatic interactions, and/or interpenetrating networks.

In one embodiment according to the second aspect of the present invention, the one or more cell-adhesion ligand(s) is a peptide comprising the amino acid sequence RGD, such as a peptide comprising the amino acid sequence GRGDSP.

In one embodiment according to the second aspect of the present invention, the biocompatible hydrogel beads comprise alginate, fibrin, gelatin, agarose, modified or unmodified poly-ethylene glycol, carrageenan, pectin or any combination thereof.

In one embodiment according to the second aspect of the present invention, the biocompatible hydrogel beads are made from alginate, fibrin, gelatin, agarose, modified or unmodified poly-ethylene glycol, carrageenan, pectin or any combination thereof.

In one embodiment according to the second aspect of the present invention, the biocompatible hydrogel beads are biocompatible alginate beads. The biocompatible alginate beads have preferably been produced by competitive ligand exchange crosslinking as described in e.g. example 1 of WO2017153947. In another preferred embodiment, the biocompatible hydrogel beads are biocompatible hydrogel beads comprising alginate. The biocompatible hydrogel beads comprising alginate have preferably been produced by competitive ligand exchange crosslinking as described in e.g. example 3 of WO2017153947.

In one embodiment according to the second aspect of the present invention, the biocompatible hydrogel beads are biocompatible alginate beads and the alginate is RGD-coupled alginate or GRGDSP-coupled alginate.

In one embodiment of the second aspect, the biocompatible hydrogel beads are biocompatible alginate beads and the means for dissolving or degrading the biocompatible hydrogel alginate beads is selected from the group consisting of alginate lyase treatment; treatment with a divalent cation chelating agent; and any combination thereof.

In one embodiment according to the second aspect of the present invention, the biocompatible hydrogel beads are biocompatible hydrogel microbeads, preferably biocompatible hydrogel microbeads of an average size less than 500 µm in their largest dimension; more preferably of an average size in the range 10 to 200 µm, such as 10 to 150 µm or 30 to 150 µm, in their largest dimension; even more preferably of an average size in the range 30 to 90 µm, such as 30 to 80 µm, in their largest dimension; and most preferably of an average size in the range 50 to 80 µm in their largest dimension. The size of the beads is measured at their swollen equilibrium size in a liquid environment of physiological ion concentration, such as cell culture medium, see section 1.2 of example 1.

In one embodiment, at least 60 %, such as at least 65 %, at least 70 %, at least 75 %, at least 80 %, at least 85 %, at least 90 %, at least 95 % or at least 99 %, of the biocompatible hydrogel beads have a size in the range 10 to 200 µm in their largest dimension, such as a size in the range 10 to 150 µm in their largest dimension, a size in the range 30 to 150 µm in their largest dimension or a size in the range 30 to 90 µm in their largest dimension. Thus, the person skilled in the art will understand that if there are 100 000 biocompatible hydrogel beads and 60 % of the biocompatible hydrogel beads have a size in the range 10 to 200 µm in their largest dimension there are in fact 60 000 biocompatible hydrogel beads which have a size in the range 10 to 200 µm in their largest dimension.

### BRIEF DESCRIPTION OF DRAWINGS

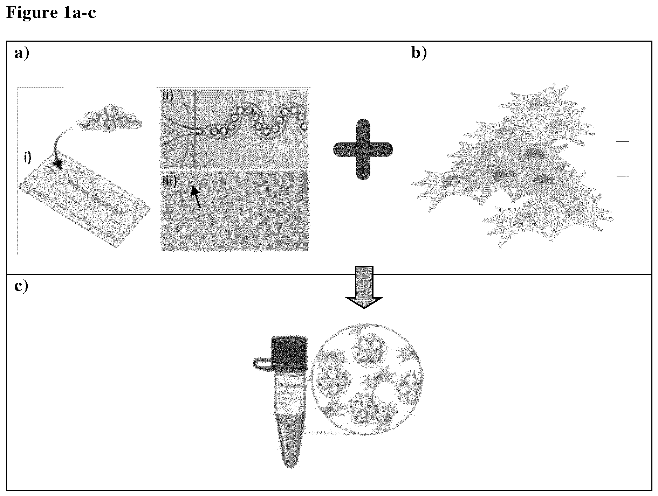

**Figure 1a****-c** is a schematic illustration of the approach used to produce bead-associated cells. RGD-functionalized alginate microbeads are produced in a microfluidic process (a) and mixed with human anchorage dependent cells (b). The human anchorage dependent cells attach to an outer surface of the RGD-functionalized alginate microbeads to obtain bead-associated cells (c).

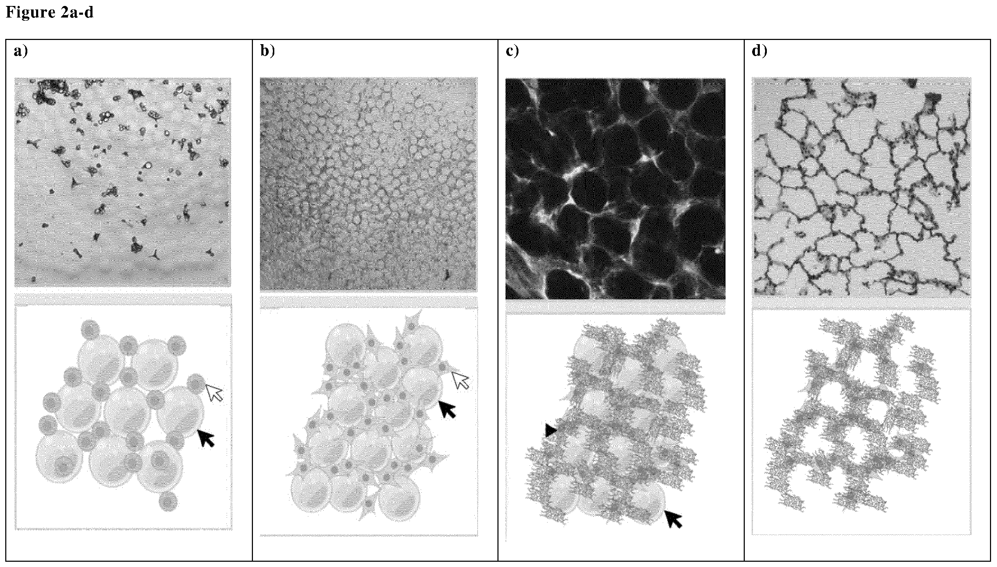

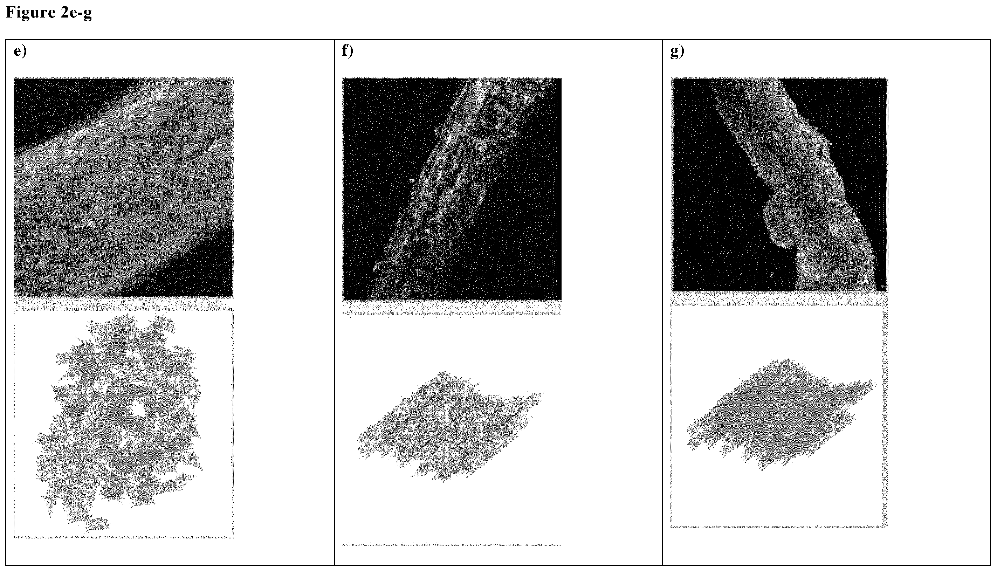

**Figure 2a****-g** illustrates the bead-associated cells of figure 1c exploited to produce extracellular matrix thereby obtaining various tissue scaffolds. Panels a-e and f-g are schematic illustrations (lower panels) and corresponding microscopy images (upper panels) of the static culture phase and the mechanical conditioning phase of the various tissue scaffolds described herein. The human anchorage dependent cells attach to the RGD-functionalized alginate microbeads and one another forming a 3D lattice structure (a), on which the cells can grow and proliferate (b). Over time the cells produce their own supportive extracellular matrix (c) that can support the structure when the microbeads are selectively removed (d). The cells continue to deposit extracellular matrix after the microbeads are removed (e). If the sample is subjected to mechanical conditioning, the extracellular matrix deposition will increase and produces aligned fibers (f). At last, the cells may be removed leaving a tissue scaffold produced only of human extracellular matrix (g). Closed arrows=microbeads; open arrows=cells; closed arrowhead=cell-secreted extracellular matrix; open arrow head=extracellular matrix with aligned fibers upon mechanical conditioning.

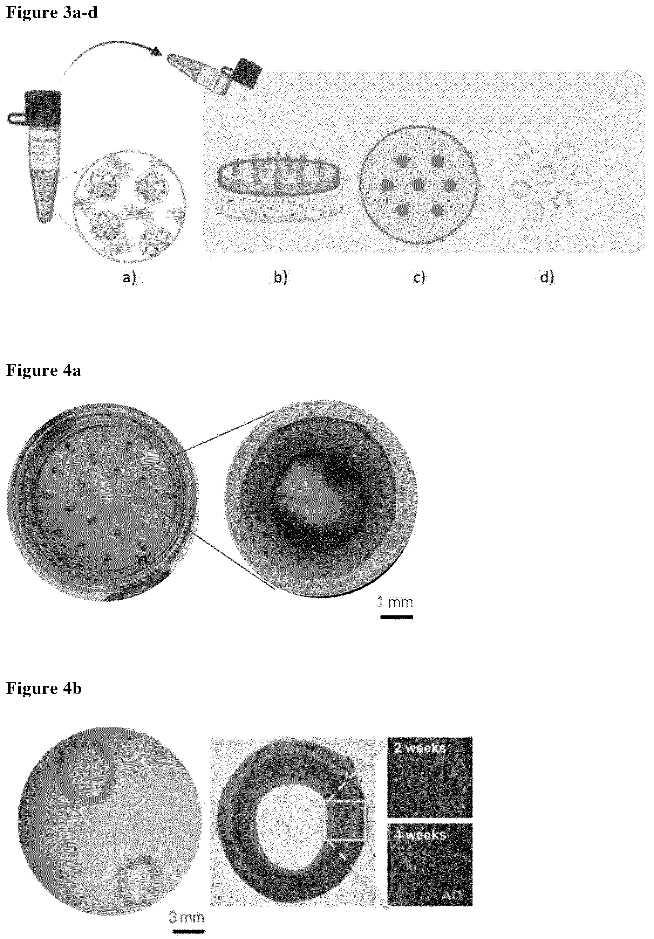

**Figure 3a****-d** is a schematic illustration of a tissue scaffold production process, wherein the tissue scaffold has the shape of a circular tube. The bead-associated cells of figure 1c (a) are cast into an agarose mold viewed from the side (b) and top (c) and form cohesive rings as the cells grow and secrete extracellular matrix. When removed from molds, the tissue scaffolds maintain their shape and high viability despite the large size of the scaffolds (d).
**Figure 4a** are photographs of tissue scaffolds having the shape of circular tubes. Once cast into the molds, the bead-associated cells of figure 1c form cohesive rings wherein the cells proliferate and grow as they deposit extracellular matrix in a ring-shaped structure. Despite the thickness of the ring edges approaching 1 mm, the tissue scaffolds remain viable throughout the construct.
**Figure 4b** are photographs and microscopy images of ring-shape tissue scaffolds. When removed from the molds depicted in figure 4a, the constructs retain their shape and high viability despite the large size of the constructs (two separate representative samples).

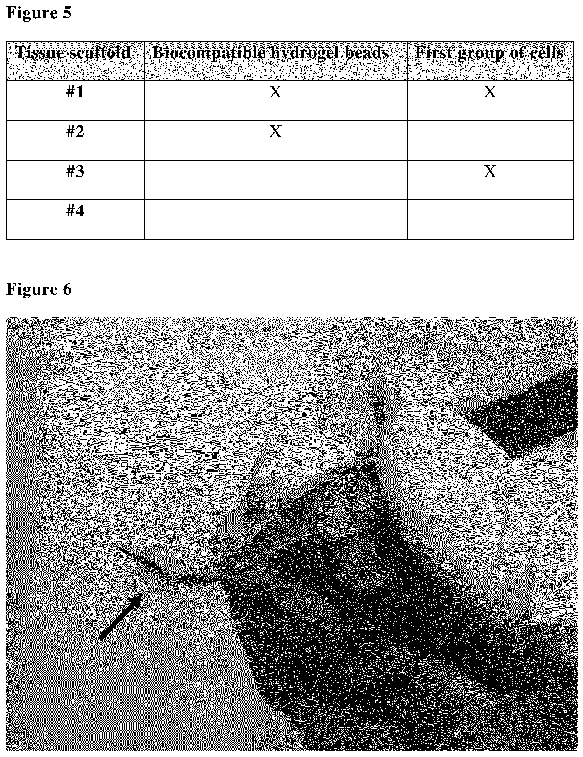

**Figure 5** is a schematic presentation of the four different tissue scaffolds referred to herein indicating presence or absence of biocompatible hydrogel beads and first group of cells.
**Figure 6** illustrates a self-supporting tissue scaffold having the shape of a ring.

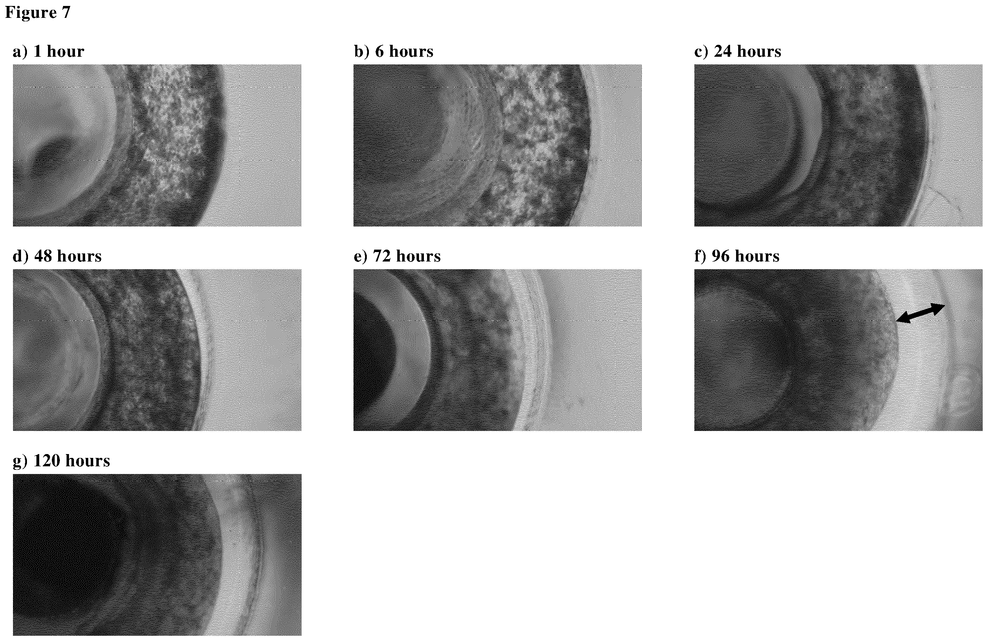

**Figure 7a****-g** are photographs of T1 tissue rings at various time points post-molding (7a, 1 hour post molding; 7b, 6 hours post molding; 7c, 24 hours post molding; 7d, 48 hours post molding; 7e, 72 hours post molding; 7f, 96 hours post molding; and 7g, 120 hours post molding). T1 tissue rings have been produced by using annular wells having 3 mm inner diameter, 1 mm thickness of wells and 3 mm depth (example 2, section 2.4). The T1 tissue rings are thinner than the T2 tissue rings. The distance between outer surface of tissue ring and surface of mold is indicated by a black arrow, where a longer distance is associated with higher degree of tissue ring contraction.

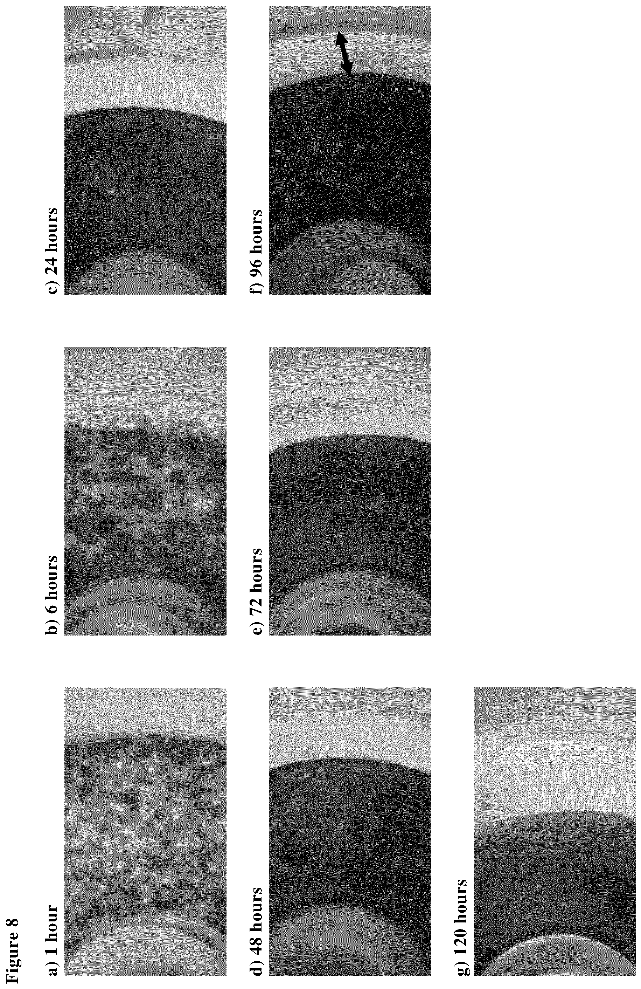

**Figure 8a****-g** are photographs of T2 tissue rings at various time points post-molding (8a, 1 hour post molding; 8b, 6 hours post molding; 8c, 24 hours post molding; 8d, 48 hours post molding; 8e, 72 hours post molding; 8f, 96 hours post molding; and 8g, 120 hours post molding). T2 tissue rings have been produced by using annular wells having 3 mm inner diameter, 2 mm thickness of wells and 4 mm depth (example 2, section 2.4). The T2 tissue rings are thicker than the T1 tissue rings. The distance between outer surface of tissue ring and surface of mold is indicated by a black arrow, where a longer distance is associated with higher degree of tissue ring contraction.

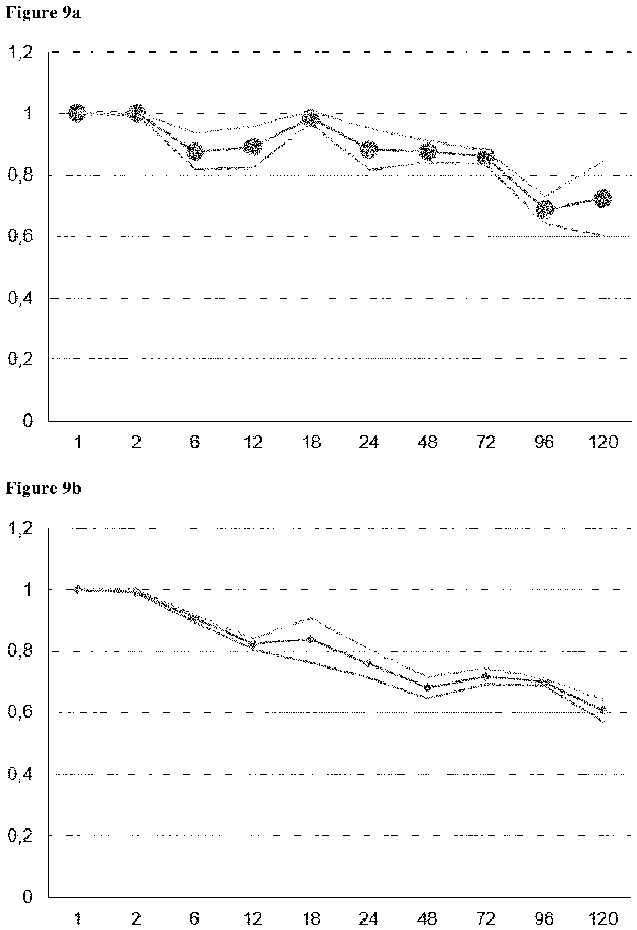

**Figure 9a****-b** illustrates contraction of the T1 and T2 tissue rings respectively over time. X-axis indicates time (hours) post molding and Y-axis indicates degree of contraction (width of tissue ring : width of mold ratio). Line with marker represent average ring width : mold width ratio at different time points and lines without marker indicating ± standard deviation. High degree of contraction, i.e. low value on the y-axis, indicating that the tissue rings are self-supporting.

### DEFINITIONS

The expression "anchorage dependent cells" as used herein are any type of cells that will grow, survive, maintain function or differentiate when attached to a surface, also known as substrate-dependent cells.

The word "repopulate" or "repopulation" as used herein covers the process of seeding any of the herein mentioned tissue scaffolds wherein the first group of cells have been removed and replaced by a second group of cells, hence allowing this new population of cells (second group of cells) to grow, survive, maintain function or differentiate on the decellularized tissue scaffold.

The word "hydrogel" as used herein is a crosslinked hydrophilic polymer being highly absorbent while still maintaining a well-defined structure. The hydrogel may be synthetic or derived from nature.

The expression "hydrogel beads" as used herein are three-dimensional cross-linked networks of hydrophilic polymers formed in a spherical shape.

The expression "extracellular matrix proteins" or "extracellular matrix" is herein interchangeably used to describe the intricate network composed of an array of multidomain macromolecules, such as collagen, elastin, enzymes, glycoproteins and hydroxyapatite, organized in a cell/tissue-specific manner.

The expression "bead-associated cells" as referred to herein is used in describing anchorage dependent cells linked to hydrogel beads through various interactions.

"Attachment of cells to hydrogel beads" and "cell adhesion" is herein interchangeably used in describing the attachment of the first group of cells to the biocompatible hydrogel beads, thus obtaining bead-associated cells.

The expression "hollow bodies" as referred to herein refers to the empty space that arise in the tissue scaffold when the biocompatible hydrogel beads are degraded or dissolved, i.e. the space that was previously occupied by a hydrogel bead.

The words "bioinert" or "inert" as used herein is interchangeably used to describe the feature of being deficient in active properties, i.e. lacking a usual or anticipated chemical or biological action.

The words "biocompatible" or "compatible" as used herein is interchangeably used to describe the characteristic of being compatible with living tissue or a living system by not being toxic, physiologically reactive or causing immunological rejection.

The term "microbead" as used herein refers to beads less than 1000 µm in their largest dimension.

### Detailed description of the invention

Unless specifically defined herein, all technical and scientific terms used have the same meaning as commonly understood by a skilled artisan in the fields of genetics, biochemistry, and molecular biology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail.

Where a numerical limit or range is stated herein, the endpoints are included. Also, all values and sub ranges within a numerical limit or range are specifically included as if explicitly written out.

Tissue engineering as a field seeks to develop biological substitutes to restore the biological function of a tissue or whole organ and comprise methods of culturing cells on a supportive matrix to form new tissues of intended shape and function. However, there are several shortcomings to the method and clinical translation of such engineered tissues are low. The tissue scaffold that serves as an intermediate in the production of a new functional tissue may present a risk of immunologic and/or inflammatory reactions when implanted. Furthermore, the tissue scaffold should be processable to form tissue scaffolds of a desired shape for the tissue of interest. Consequently, there is a need in the art to develop techniques that improve the tissue scaffolds for use in tissue engineering.

The present inventors have solved the above needs by providing improved manufacturing methodologies which enables production of complex three-dimensional tissue scaffolds. These tissue scaffolds are essentially non-immunogenic when implanted into a human, mimic native extracellular matrix and may be repopulated and modulated by host cells thereby becoming living tissues.

In general, the present invention involves the production of an engineered tissue scaffold developed primarily by *in vitro* culturing of a first group of cells adhered to biocompatible hydrogel beads and secreting extracellular matrix. Upon removal of the biocompatible hydrogel beads and decellularization of the first group of cells, a tissue scaffold comprising extracellular matrix of a desired shape is provided. The decellularized tissue scaffold may further be repopulated and *in vitro* cultured with a second group of cells characteristic of the intended tissue or organ to be replaced or augmented. However, also within the scope of the invention, the decellularized tissue scaffold may be implanted into a human or animal wherein the recipients' cells may infiltrate and repopulate the decellularized tissue scaffold *in vivo.* Hence, within the scope of the present invention are engineered tissue scaffolds cultured primarily *in vitro* and in certain embodiments *in vivo.*

In general, for the tissue scaffold product to be safe to implant into a human the reagents used throughout the method, such as the hydrogel beads, are preferably biocompatible, degradable or dissolvable and/or possible to remove, e.g. washed away, before the implantable product is ready for use.

Thus, according to one aspect, the present invention provides a method of manufacturing a tissue scaffold, the method comprising the following steps:
a) providing a first group of cells and biocompatible hydrogel beads;
b) bringing the first group of cells together with the biocompatible hydrogel beads to obtain a first cell-bead mixture;
c) culturing the first cell-bead mixture to produce extracellular matrix thereby obtaining a first tissue scaffold;
d) optionally, subjecting the first tissue scaffold to mechanical conditioning thereby obtaining a mechanically conditioned first tissue scaffold;
wherein
- the first group of cells are anchorage dependent cells that can produce and secrete extracellular matrix;
- the biocompatible hydrogel beads support attachment of the first group of cells; and
- the first group of cells and the biocompatible hydrogel beads of step b) are brought together in a mold to obtain the first cell-bead mixture; or
the first cell-bead mixture of step b) is incubated to allow formation of bead-associated cells and then transferred into a mold.

### The first group of cells

The **first step** of the method refers to a first group of cells. This group of cells are anchorage dependent cells used to seed the hydrogel beads and preferably secret as much extracellular matrix as possible to build a first tissue scaffold that holds its shape without the support of an additional substrate.

The first group of cells may be derived from an animal or human donor or from an established cell line. Preferably, the cells are of the same species as the intended recipient, e.g. to reduce immunologic reactions and increase compatibility of the tissue scaffold with recipient. Rigorous screening of the cells for transmissible diseases (e.g., HIV or hepatitis), further decrease the infectious risk associated with the tissue scaffold products. The cells may further have desirable properties such as an ability to grow well in culture, or the cells are genetically modified to alter their secretion of extracellular matrix components.

The first group of cells are in principle any type of cells that will grow, survive, maintain function or differentiate when attached to a surface, also known as anchorage-dependent cells. Additionally, according to the present method, the first group of cells can produce and secrete extracellular matrix proteins. Preferably, the first group of cells produce as much extracellular matrix as possible. Thus, in one embodiment according to the present invention, the first group of cells are selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells and in particular adipose tissue derived mesenchymal stem cells, or any combination thereof. In a preferred embodiment, the first group of cells is adipose tissue derived mesenchymal stem cells. In one embodiment the first group of cells have been genetically modified to increase their secretion of extracellular matrix.

### Hydrogel beads and bead-associated cells

According to the **second step** of the method, the first group of cells is brought together with biocompatible hydrogel beads to facilitate attachment of the first group of cells to the biocompatible hydrogel beads and thereby obtain bead-associated cells. Thus, the biocompatible hydrogel beads are the initial substrate for the first group of cells to grow on when producing a tissue scaffold of a desired shape for a tissue or organ of interest.

A person skilled in the art will easily understand the conditions needed to achieve attachment and/or growth of the cells on the hydrogel beads. The first group of cells and the biocompatible hydrogel beads may be incubated or cultivated in a still/static, shaking or rotating incubator in a tissue culture flask or any other form of sterile container. Further reference is made to section 1.3 of Example 1.

For the first group of cells to attach to the biocompatible hydrogel beads, the biocompatible hydrogel beads need to support attachment of the first group of cells. If the biocompatible hydrogel beads do not support attachment of the first group of cells, one or more cell-adhesion ligand(s) may be incorporated into the biocompatible hydrogel beads or coated on the surface of the biocompatible hydrogel beads to achieve proper conditions for attachment, herein also called functionalization of the biocompatible hydrogel beads. For example, covalently conjugated biocompatible hydrogel beads with heparin binding peptides or peptide sequences found in extracellular matrix proteins can be used to tailor the type and degree of interaction with cells. Interaction of cells with biocompatible hydrogel beads comprising cell-adhesion ligand(s) is often mediated through cellular receptors on the surface of the cells recognizing the cell adhesion molecules at the surface of the hydrogel.

Thus, in one embodiment according to the present invention, the biocompatible hydrogel beads have one or more cell-adhesion ligand(s) on their outer surface.

Cells, in particular anchorage dependent cells, do not readily attach and grow on or within alginate hydrogels. However, free hydroxyl and carboxyl groups are distributed along the backbone of alginate, rendering alginate suitable for chemical modification, e.g. covalently conjugating alginate with cell-adhesion ligand(s).

According to one embodiment according to the present invention, the biocompatible hydrogel beads are alginate beads comprising one or more cell-adhesion ligand(s) on the outer surface of said alginate beads. The one or more cell-adhesion ligand(s) may e.g. be i) a peptide comprising a cell adhesive amino acid sequence, such as RGD, YIGSR, GFOGER or PHSRN; or ii) a charged polymer, such as poly-L-Lys. The one or more cell-adhesion ligand(s) preferably being incorporated into the biocompatible hydrogel beads via covalent tethering, electrostatic interactions, and/or interpenetrating networks. Peptide coupled alginates are e.g. available from Novamatrix^{®} (https://novamatrix.biz/).

Functionalization of the biocompatible hydrogel beads enables not only attachment of one cell to one bead, but various attachment of one or more cells to one or more beads, cell to cell attachment and/or attachment between cells, beads and the extracellular matrix forming a three-dimensional lattice structure. Thus, in one embodiment according to the present invention, neighboring biocompatible hydrogel beads are connected to one another by a network of the first group of cells and the extracellular matrix.

Hydrogels, like alginate, are optimal substrates for biomedical applications as they are crosslinked hydrophilic polymers that themselves do not dissolve in water, and highly absorbent while still maintaining a well-defined structure. This is a beneficial characteristic when serving as a substrate to culture cells as the hydrogel itself may serve as a reservoir for nutrients deep within the tissue scaffold.

Further, the spacing between the biocompatible hydrogel beads creates a porous structure which allows for diffusion of nutrients throughout the tissue scaffold as the cells grow and secret extracellular matrix. This provides an advantage over bulk gels used in prior art in that also tissue scaffolds of greater thickness can be produced without re-seeding the biocompatible hydrogel beads to obtain or maintain a live cell population that produce extracellular matrix. The extended ability for nutrient diffusion by using biocompatible hydrogel beads according to the present invention therefore provides the advantage of enabling production of complex three-dimensional tissue scaffolds.

The person skilled in the art will acknowledge that the size of the biocompatible hydrogel beads likely will affect i) the porosity of the structure, i.e. spacing between the biocompatible hydrogel beads; and ii) the surface area to volume ratio of the beads. While increased bead size would be expected to increase the volume of the spacing between the beads, the surface area to volume ratio of the beads would be expected to decrease. Thus, increasing the size of the beads would likely improve diffusion of nutrients throughout the structure but at the same time cause less efficient production of extracellular matrix. Thus, the size of the biocompatible hydrogel beads should be selected for optimal nutrient diffusion and optimal number of cells per volume.

In one embodiment, the biocompatible hydrogel beads are biocompatible hydrogel microbeads, preferably biocompatible hydrogel microbeads of an average size less than 500 µm in their largest dimension; more preferably of an average size in the range of 10 to 500 µm, such as 10 to 200 µm, such as 10 to 150 µm or 30 to 150 µm, in their largest dimension; even more preferably of an average size in the range 30 to 90 µm, such as 30 to 80 µm, in their largest dimension; and most preferably of an average size in the range 50 to 80 µm in their largest dimension.

In one embodiment, at least 60 %, such as at least 65 %, at least 70 %, at least 75 %, at least 80 %, at least 85 %, at least 90 %, at least 95 % or at least 99 %, of the biocompatible hydrogel beads have a size in the range 10 to 200 µm in their largest dimension, such as a size in the range 10 to 150 µm in their largest dimension, a size in the range 30 to 150 µm in their largest dimension or a size in the range 30 to 90 µm in their largest dimension. Thus, the person skilled in the art will understand that if there are 100 000 biocompatible hydrogel beads and 60 % of the biocompatible hydrogel beads have a size in the range 10 to 200 µm in their largest dimension there are in fact 60 000 biocompatible hydrogel beads which have a size in the range 10 to 200 µm in their largest dimension.

As previously discussed, hydrogels are highly absorbent and the size of the beads is therefore measured at their swollen equilibrium size in a liquid environment of physiological ion concentration, such as cell culture medium. Section 1.2 of example 1 provides a technique for measuring the diameter of the hydrogel beads.

Biocompatible hydrogels may be produced by any method of cross-linking polymers such as free-radical polymerization, chemical reaction or ionizing radiation. In the case of divalent-ion cross-linking hydrogels, it is preferred that the biocompatible hydrogel is prepared by competitive ligand exchange as described in WO2017153947, in particular example 1 or example 3 of said document.

In one embodiment, the biocompatible hydrogel beads of the present invention are made from or comprise alginate; fibrin; gelatin; agarose; modified or unmodified poly-ethylene glycol; carrageenan; pectin; hyaluronic acid; collagen; cellulose derivatives, such as e.g. hydroxy methyl propyl cellulose (HPMC) or methyl propyl cellulose (MPC); or any combination thereof. Preferably, the polymers used in production of the biocompatible hydrogel beads are degradable or dissolvable and preferably have inherently low immunogenicity. Preferably, the hydrogel beads are prepared from natural polymers.

According to one embodiment, the biocompatible hydrogel beads are either alginate beads or beads comprising alginate.

Alginate have been used for many biomedical applications due to its biocompatibility, low toxicity, and ease of gelation by addition of divalent cations such as e.g. Ca²⁺. Alginate hydrogels can be prepared by various cross-linking methods well known to the skilled person, including competitive ligand exchange as discussed above, from a variety of commercially available alginate, typically obtained from brown algae, such as e.g. *Laminaria hyperborean, Laminaria digitatat, Laiminaria japonica, Ascopyllum nodosum* or *Macrocystis pyrifera.*

Alginate is a naturally occurring anionic polymer containing blocks of (1,4)-linked β-D-mannuronate (M) and α-L-guluronate (G) residues. The blocks are composed of consecutive G residues (GGGGGG), consecutive M residues (MMMMMM), and alternating M and G residues (GMGMGM). Alginates extracted from different sources differ in M and G contents as well as the length of each block, and more than 200 different alginates are currently being manufactured

Only the G-blocks of alginate are believed to participate in intermolecular cross-linking with divalent cations (e.g., Ca²⁺) to form hydrogels. The composition (i.e., M/G ratio), sequence, G-block length, and molecular weight are thus factors affecting the physical properties of alginate and its resultant hydrogels.

The mechanical properties of alginate gels typically are enhanced by increasing the length of G-block and molecular weight. However, an alginate solution formed from high molecular weight polymer also becomes highly viscous. Manipulation of the molecular weight and its distribution can independently control the pre-gel solution viscosity and post-gelling stiffness. The elastic modulus of gels can be increased significantly, while the viscosity of the solution minimally raised, by using a combination of high and low molecular weight alginate polymers.

The skilled person will acknowledge that that the proportions of M and G residues varies dependent upon the species of brown alga from which the alginate is obtained. The skilled person will also acknowledge that some alginate having high proportions of G-residues may commonly be referred to as G-rich alginate, whereas alginates having low proportions of G-residues may commonly be referred to as M-rich alginate.

In one embodiment, the biocompatible hydrogel beads used in the present method are made of alginate having high proportions of G-residues, i.e. G-rich alginate. According to one embodiment, the biocompatible hydrogel beads used in the present method are made of alginate comprising ≥ 30% G-residues, such as ≥ 35% G-residues, ≥ 40% G-residues, ≥ 45% G-residues, ≥ 50% G-residues, ≥ 55% G-residues, ≥ 60% G-residues, ≥ 65% G-residues or ≥ 70% G-residues (monomer units). In a preferred embodiment, the biocompatible hydrogel beads used in the present method are made of alginate comprising ≥ 40% G-residues, such as ≥ 45% G-residues (monomer units).

In one embodiment, the biocompatible hydrogel beads used in the present method are made of a mixture of at least two different alginates, wherein each of the alginates independently comprises ≥ 30% G-residues, such as ≥ 35% G-residues, ≥ 40% G-residues, ≥ 45% G-residues, ≥ 50% G-residues, ≥ 55% G-residues, ≥ 60% G-residues, ≥ 65% G-residues or ≥ 70% G-residues (monomer units).

In another embodiment, the biocompatible hydrogel beads used in the present method are made of alginate comprising 30-90 % G-residues, such as 30-80 % G-residues, 40-80 % G-residues or 40-75 % G-residues (monomer units).

In one embodiment, the biocompatible hydrogel beads used in the present method are made of a mixture of at least two different alginates, wherein each of the alginates independently comprises 30-90 % G-residues, such as 30-80 % G-residues, 40-80 % G-residues or 40-75 % G-residues (monomer units).

In another embodiment, the biocompatible hydrogel beads used in the present method are made of alginate with a G:M ratio ≥ 0.4, such as a G:M ratio ≥ 0.5, a G:M ratio ≥ 0.7, a G:M ratio ≥ 0.9, a G:M ratio ≥ 1, a G:M ratio ≥ 1.2, a G:M ratio ≥ 1.4 or a G:M ratio ≥ 1.5.

In one embodiment, the biocompatible hydrogel beads used in the present method are made of a mixture of at least two different alginates, wherein each of the alginates independently has a G:M ratio ≥ 0.4, such as a G:M ratio ≥ 0.5, a G:M ratio ≥ 0.7, a G:M ratio ≥ 0.9, a G:M ratio ≥ 1, a G:M ratio ≥ 1.2, a G:M ratio ≥ 1.4 or a G:M ratio ≥ 1.5.

In one embodiment, the biocompatible hydrogel beads used in the present method are made of alginate with a molecular weight in the range of 10-300 kDa, such as 10-250 kDa, 10-200 kDa, 10-150 kDa, 10-100 kDa, 50-300 kDa, 50-250 kDa, 50-200 kDa, 50-150 kDa or 50-100 kDa.

In one embodiment, the biocompatible hydrogel beads used in the present method are made of a mixture of at least two different alginates, wherein each of the alginates independently has a molecular weight in the range of 10-300 kDa, such as 10-250 kDa, 10-200 kDa, 10-150 kDa, 10-100 kDa, 50-300 kDa, 50-250 kDa, 50-200 kDa, 50-150 kDa or 50-100 kDa.

### Mold

According to the first aspect of the present invention, the first group of cells and the biocompatible hydrogel beads of step b) are brought together in a mold to obtain the first cell-bead mixture; or the first cell-bead mixture of step b) is incubated to allow formation of bead-associated cells and then transferred into a mold.

Transferring the bead-associated cells, according to the present method, may be any procedure that physically move the bead-associated cells from one container to the next, such as pouring or pipetting.

In context of the present invention, the mold may be made of a bioinert material and functions as support for the first cell-bead mixture until the tissue scaffold can hold its shape by itself. Hence, in one embodiment, the mold has one or more of the following properties: (1) allow control of geometry; (2) prevent cell attachment or infiltration; and (3) allow exchange of nutrients and gases between exterior and interior of the mold.

The mold may be made of any inert and biocompatible material with the above characteristics. For example, the mold material should be selected in order to allow for precise control over construct shape and dimensions, and that can be removed from the tissue scaffold either mechanically (e.g. pull-out) or chemically (via degradation). The mold may according to one embodiment be made of co-polymers of lactic and glycolic acid (PLGA or PDLG), polycaprolactones, carbohydrate glasses, inert Gore-Tex material typically used in e.g. vascular grafts, ePTFE (expanded polytetrafluoroethylene), PTFE (polytetrafluoroethylene), PDMS (Polydimethylsiloxane), silicone, PEEK (Polyether ether ketone) and/or agarose. According to one embodiment, the mold used in the present method is agarose.

Agarose is widely used as a mold material for fabricating self-assembled tissues due to its biocompatibility, permeability, and non-cell adhesive properties. For example, agarose molds may be obtained by 3D printing, formed into the desired form of the tissue scaffolds prepared according the present method. Reference may e.g. be made to Strobel et al. (2018), J. Vis. Exp., 134, pp. 55618.

In one embodiment, the mold is completely or partly submerged in cell culture media. In one embodiment, the mold has the shape of an annular well.

The mold, in the context of the present invention, provides exterior support for the first cell-bead mixture to form a lattice of desired shape for the intended tissue or organ. Preferably, the mold allows to produce tissue scaffolds of any desired shape and/or complex geometry.

### Culturing the first cell-bead mixture to produce ECM

According to step c) of the method according to the first aspect of the present invention, the first cell-bead mixture is cultured in the mold to produce extracellular matrix, thereby obtaining a first tissue scaffold. The extracellular matrix formed will take the shape of the mold used.

Conditions and culture media suitable for culturing the first cell-bead mixture prior to or after transfer to the mold, as well as for culturing the cells present in the scaffold(s) of the present invention are known to a person skilled in the art. Suitable culture media and supplements for culturing anchorage dependent cells, such as the cells of the first group of cells, applicable in the present method are available from various providers well known to the skilled person, such as e.g. from ThermoFisherScientific or PromoCell. In a preferred embodiment, the first group of cells produce and secret as much extracellular matrix proteins as possible as they grow and divide on the biocompatible hydrogel beads.

Furthermore, various growth conditions can be selected to enhance the process of extracellular matrix production, and/or stimulate the development of desirable mechanical, physical, or biochemical properties. Such growth conditions may include the use of growth media supplemented with amino acids such as proline, alanine, glycine and/or ascorbic acid to enhance extracellular matrix protein production. Additionally, the growth media may be supplemented with growth factors such as FGF2 (fibroblast growth factor) and/or any other growth factor that may be important for control of extracellular matrix production and degradation and the interaction between cells and extracellular matrix. In one embodiment, the media is supplemented with amino acids according to Section 1.3 of Example 1 and/or growth factors according to Section 1.1 of Example 1. In one embodiment, the first cell-bead mixture is cultured in the mold in the presence of one or more agents which stimulates the cells of the first cell-bead mixture to produce and/or secrete extracellular matrix. The one or more agents which stimulates the cells to produce and/or secrete extracellular matrix is selected from the group consisting of proline, glycine, alanine, ascorbic acid and any combination thereof.

The first cell-bead mixture is cultured in the mold to produce extracellular matrix at least until the first tissue scaffold can be removed from the mold and still maintain its shape, i.e. that the first tissue scaffold is self-supporting. Example 2 provides methodology which may be used to decide when a tissue scaffold is self-supporting.

Culturing until the first tissue scaffold can be removed from the mold and still maintain its shape will typically range from 2 days to 2 weeks depending on the type of cells used, the mold, and the experimental conditions. Example 2 provides methodology which may be used to decide when a tissue scaffold is self-supporting.

### Mechanical conditioning

The first-, second-, third- and/or fourth tissue scaffold may be subjected to mechanical conditioning thereby obtaining a more suitable tissue architecture and an extracellular matrix environment that more closely mimics native tissue. Clinical outcomes may be improved by mechanically conditioning the tissue scaffolds to enhance their properties such as expression of desired cell phenotypes, altered tensile strength and alignment of fibers with the load applied mimicking the load naturally exposed to within the body.

Mechanical conditioning of any tissue scaffold according to the present invention can be performed by various devices known in the art capable of applying mechanical forces to the cells (e.g. stretch) without damaging the tissue scaffold (e.g. Strobel, Tis Eng Part A 2018; J Tissue Eng Regen Med 10, E204, 2016). Such devices may include various types of bioreactor systems or thermally responsive polymers that create an elastic substrate for the cells, and the mechanical forces may be, but is not limited to, air or water pressure or mechanical and/or thermal stretching.

If the tissue scaffold has the shape of a tube, the tissue scaffold may e.g. be subjected to mechanical conditioning selected from the group consisting of:
- cyclic strain and/or radial distention on the interior of the scaffold, preferably applied via pressurized fluid or air flow; such as cyclic circumferential strain and/or radial distention on the interior of the scaffold, preferably applied via pressurized fluid or air flow;
- axial strain, preferably applied via extension of the opposing ends of the scaffold;
- compressive strain, preferably applied from one side of the scaffold;
- fluid shear stress over or through the scaffold; or
- any combination thereof.

### Removal of the biocompatible hydrogel beads

In one embodiment according to the present invention, the biocompatible hydrogel beads are subjected to means for dissolving or degrading the biocompatible hydrogel beads thereby obtaining a third tissue scaffold.

The skilled person will acknowledge that the hydrogel beads may be removed by well-known techniques to the skilled person. The method for dissolving or degrading a hydrogel depends upon the hydrogel in questions.

In one embodiment, the biocompatible hydrogel beads are biocompatible alginate beads and the means for dissolving or degrading the biocompatible hydrogel alginate beads is selected from the group consisting of alginate lyase treatment; treatment with a divalent cation chelating agent; and any combination thereof.

In one embodiment according to the present invention, the biocompatible hydrogel beads are self-degradable or self-dissolvable. The terms "self-degradable" and "self-dissolvable" means that the biocompatible hydrogel bead will degrade or dissolve by itself. Thus, there is no need for adding any agents for the degradation or dissolution to take place. The time needed for the degradation or dissolution to be completed will typically be decreased at increasing temperature.

Immediately after the biocompatible hydrogel beads have been removed, there will be hollow bodies that previously were occupied by the biocompatible hydrogel beads. In one embodiment according to the present invention, those hollow bodies are reconstituted by i) secretion of extracellular matrix within the hollow bodies; and/or ii) facilitating migration of the first group of cells into the hollow bodies; and/or allow contractile forces of the first group of cells to close the hollow bodies. It is preferred that the hollow bodies are filled with extracellular matrix and/or first group of cells prior to mechanical conditioning and/or decellularization.

### Decellularization in general

The present invention further involves subjecting the first- or third- tissue scaffold to decellularization thereby obtaining a second- or fourth- tissue scaffold respectively.

Decellularization, at any step according to the present invention, is any techniques that remove cellular component while leaving the extracellular matrix secreted by the first group of cells substantially intact.

In one embodiment according to the present invention, decellularization is selected from the group consisting of physical treatment, chemical treatment, enzymatic treatment, and any combination thereof.

### Decellularization - Physical treatment

The most common physical treatments used to lyse, kill, and remove cells from an extracellular matrix of a tissue scaffold are through the use of temperature, pressure, and electrical disruption. Thus, in one embodiment, decellularization is achieved through the use of temperature, pressure and/or electrical disruption.

Temperature methods are often used in a rapid freeze-thaw mechanism. By quickly freezing a tissue, microscopic ice crystals form around the plasma membrane and the cell is lysed. Thus, in one embodiment decellularization is achieved using rapid freeze-thaw mechanism.

Pressure decellularization involves the controlled use of hydrostatic pressure applied to a tissue scaffold. This is done best at high temperatures to avoid unmonitored ice crystal formation that could damage the scaffold. Thus, in one embodiment decellularization is achieved by use of hydrostatic pressure applied to the tissue scaffold.

Electrical disruption of the plasma membrane is another option to lyse cells housed in a tissue scaffold. By exposing a tissue scaffold to electrical pulses, micropores are formed at the plasma membrane. The cells eventually turn to death after their homeostatic electrical balance is ruined through the applied stimulus. Thus, in one embodiment decellularization is achieved by exposing the tissue scaffold to electrical pulses thereby forming micropores at the plasma membrane of the cells.

In one embodiment decellularization is achieved by using rapid freeze-thaw mechanism, use of hydrostatic pressure applied to the tissue scaffold, exposing the tissue scaffold to electrical pulses or any combination thereof.

After lysing the cells, the scaffold can preferably be further exposed to liquidized chemicals that degrade and wash out the undesirable components.

### Decellularization - Chemical treatment

The proper combination of chemicals is selected for decellularization depending on the thickness, extracellular matrix composition, and intended use of the tissue scaffold. The chemicals typically used to kill and remove the cells include acids, alkaline treatments, ionic detergents, non-ionic detergents, and zwitterionic detergents. Thus, in one embodiment decellularization is achieved by exposing the tissue scaffold to acid(s), alkaline, ionic detergents, non-ionic detergents, and/or zwitterionic detergents.

The ionic detergent, sodium dodecyl sulfate (SDS), is commonly used because of its high efficacy for lysing cells without significant damage to the extracellular matrix. Thus, in one embodiment decellularization is achieved by exposing the tissue scaffold to SDS.

The most well-known non-ionic detergent is Triton X-100, which is popular because of its ability to disrupt lipid-lipid and lipid-protein interactions. Triton X-100 does not disrupt protein-protein interactions, which is beneficial to keeping the ECM intact. Thus, in one embodiment decellularization is achieved by exposing the tissue scaffold to Triton X-100.

EDTA is a chelating agent that binds calcium, which is a necessary component for proteins to interact with one another. By making calcium unavailable, EDTA prevents the integral proteins between cells from binding to one another. EDTA is often used with trypsin, an enzyme that acts as a protease to cleave the already existing bonds between integral proteins of neighboring cells within a tissue. Together, the EDTA-Trypsin combination makes a good team for decellularizing tissues. Thus, in one embodiment decellularization is achieved by exposing the tissue scaffold to EDTA, more preferably an EDTA-trypsin combination.

Thus, in one embodiment decellularization is achieved by exposing the tissue scaffold to SDS, exposing the tissue scaffold to Triton X-100, exposing the tissue scaffold to EDTA, exposing the tissue scaffold to EDTA-trypsin combination or any combination thereof.

After lysing the cells, the scaffold can preferably be further exposed to liquidized chemicals that degrade and wash out the undesirable components.

Decellularization by chemical treatment is the preferred decellularization option according to the present invention.

### Decellularization - Enzymatic treatment

Enzymes used in decellularization treatments are used to break the bonds and interactions between nucleic acids, interacting cells through neighboring proteins, and other cellular components. Lipases, thermolysin, galactosidase, nucleases, and trypsin have all been used in the removal of cells. After a cell is lysed with a detergent, acid, physical pressure, etc., endonucleases and exonucleases can begin the degradation of the genetic material.

Thus, in a preferred embodiment decellularization is achieved by chemical or physical treatment followed by or in combination with an enzymatic treatment. In one embodiment, the enzymatic treatment involves exposing the tissue scaffold to an enzyme selected from the group consisting of lipases, thermolysin, galactosidase, nuclease, trypsin and any combination thereof.

According to one embodiment, decellularization is achieved by exposing the tissue scaffold to at least one detergent and at least one nuclease, more preferably by exposing the tissue scaffold to a detergent selected from the group consisting of sodium dodecyl sulphate (SDS), sodium deoxycholate (SDC), Triton X and 3-[(3-Cholamidopropyl)dimethyl ammonio]-1-propanesulfonate (CHAPS), followed by exposing the tissue scaffold to nuclease treatment, such as DNase treatment.

The decellularized tissue scaffold, according to any step of the present invention, retains substantially the same shape as prior to decellularization. Thus, treatments that remove cells while causing little damage to the extracellular matrix are preferable. At any step according to the present invention the decellularized tissue scaffold is thoroughly washed to remove residual decellularization solution that may reduce biocompatibility or inhibit subsequent growth of cells on or in the following tissue scaffold.

### Second group of cells

The present invention further involves repopulating the decellularized second or fourth tissue scaffold with a second group of cells.

The second group of cells refer to the cells used to repopulate a tissue scaffold, preferably after the first group of cells have been removed by decellularization. The second group of cells may be the same or different from the first group of cells, or a group of cells having characteristic of the intended tissue or organ to be replaced or augmented.

The second group of cells may be derived from an animal or human donor or from an established cell line. Preferably, the cells are of the same species as the intended recipient, e.g. to reduce immunologic reactions and increase compatibility of the tissue scaffold with recipient. Rigorous screening of the cells for transmissible diseases (e.g., HIV or hepatitis), further decrease the infectious risk associated with the tissue scaffold products. The cells may further have desirable properties such as an ability to grow well in culture, or the cells are genetically modified to alter their secretion of extracellular matrix components.

The second group of cells herein, may be anchorage dependent cells that are the same or different to the first group of cells used to seed the biocompatible hydrogel beads (the initial substrate). In one embodiment, the second group of cells may be any type of cell suitable for reproducing a functional tissue or organ. For example, the cells used to seed the decellularized tissue scaffold, i.e. the second- or fourth-tissue scaffold, may be of a different cell type than the cells that were used to produce the decellularized tissue scaffold.

The second group of cells preferably have the ability to attach to, grow and/or differentiate when subjected to the second- or fourth- tissue scaffold according to the present invention.

In one embodiment, the second group of cells is selected from the group consisting of multipotent stem cells, pluripotent stem cells, differentiated primary mesenchymal cells, such as fibroblasts and/or smooth muscle cells, preferably human primary mesenchymal cells, such as e.g. mesenchymal stem cells and in particular adipose tissue derived mesenchymal stem cells. In one embodiment, the second group of cells is anchorage dependent cells that have been genetically modified to increase their secretion of extracellular matrix.

In a preferred embodiment, the second group of cells is progenitor cells or adult stem cells (ASCs) that are allowed to differentiate within the tissue scaffold to develop into the desired tissue or organ. Cells applicable as second group of cells, are available from various cell culture providers, cf. e.g. the 3D cell culture handbook of ThermoFisherScientific, https://assets.thermofisher.com/TFS-Assets/BID/Handbooks/3d-cell-culture-handbook.pdf , page 18; or Lonza Bioscience Solutions.

Preferably, the second group of cells are of the same species as the intended recipient and most preferably the second group of cells are derived from the intended recipient. Preferably, the second group of cells are of a cell type characteristic for the quality/features intended for the tissue or organ that the tissue scaffold is intended to replace or augment. For example, if the tissue scaffold is to be implanted into a human, the second group of cells herein may preferably be human cells. Moreover, if the tissue scaffold is used to support the development of a blood vessel for example, the second group of cells herein preferably include endothelial cells and/or smooth muscle cells.

In the most preferred embodiment, the second group of cells is endothelial cells.

According to one embodiment, the second tissue scaffold is repopulated with a second group cells. According to one embodiment, the fourth tissue scaffold is repopulated with a second group cells.

The skilled person will acknowledge that the culturing time for repopulating a decellularized second or fourth tissue scaffold according to the present invention may depend upon the size and density of the scaffold and the desired cellular structure to be obtained.

The tissue scaffold may be treated in a variety of ways either before or after seeding, such as agents selected to enhance the adherence or growth of the cells may be applied to the tissue scaffold.

Moreover, for the purpose of repopulating the decellularized second or fourth tissue scaffold, various growth conditions can be selected to enhance the process. The skilled person will acknowledge that various growth condition can be used, and that that said conditions may vary dependent upon the selected cells used for repopulation of the said scaffold(s). Various growth conditions can be selected to stimulate the development of desirable mechanical, physical or biochemical properties and/or to stimulate migration of cells into the tissue scaffold. Such growth conditions may further include the use of supplemented growth media and/or the application of mechanical, electrical and/or chemical stimuli. Thus, in general, the decellularized tissue scaffold may be subjected to any of the tissue engineered steps according to the present invention in production of a tissue scaffold.

Having generally described this invention, a further understanding can be obtained by reference to certain specific examples, which are provided herein for purposes of illustration only, and are not intended to be limiting unless otherwise specified.

### EXAMPLES

### Example 1: Production of ring-shaped tissue scaffold

### 1.1 Tissue culture

Adipose tissue derived mesenchymal stem cells (AT-MSC, Lonza) were cultured in a humidity-controlled incubator at 37°C, 5% CO₂ and supplemented with DMEM/F12 Glutamax growth medium (10% AB-serum, 1% Pen-Strep, 5 ng/ml FGF2). Cells were limited to passage less than 10 and sub-cultured prior to full confluency and use in tissue constructs.

### 1.2 Production of hydrogel microbeads

Alginate microbeads were produced using a 2-stream flow focusing droplet chip (Wunderlichips, Switzerland) with a 50x50 µm droplet forming nozzle (figure 1a). The continuous phase consisted of Novec 7500 fluorinated oil (3M, US) containing 1% Pico-Surf (Sphere Fluidics, UK) as a surfactant.

Two dispersed phases were used: (1) 1.0% (wt) total alginate consisting of 0.5% NOVATACH^{™} VLVG 4GRGDSP (4GRGDSP-coupled high G low MW alginate, G/M Ratio ≥ 1.5, nominal viscosity 10-80 mPa.s, 69% guluronate monomer units, 33 kDa) and 0.5% PRONOVA^{™} UP LVG (20-200 mPa.s, 70% guluronate monomer units, 108 kDa), containing 80 mM CaEDTA and 20 mM HEPES at pH 7.0 and (2) 1.0% (wt) total alginate consisting of 0.5% NOVATACH^{™} VLVG 4GRGDSP (4GRGDSP-coupled high G low MW alginate, G/M Ratio ≥ 1.5, nominal viscosity 10-80 mPa.s, 69% guluronate monomer units, 33 kDa) and 0.5% PRONOVA^{™} UP LVG (20-200 mPa.s, 70% guluronate monomer units, 108 kDa) containing 80 mM ZnEDDA and 20 mM HEPES at pH 7.0.

The two aqueous phases meet in a co-flow region in the microfluidic channels prior to droplet break-up. The flow rates were set to 1000 µL h⁻¹ for the continuous phase and 200 µL h⁻¹ for both aqueous phases by controlled injection using BD plastic syringes with PE plastic tubing (Scientific Commodities Inc.) mounted on syringe pumps (Harvard Apparatus, PHD ULTRA).

The microbeads were collected, and surfactant removed by adding perfluoro octanol (PFO, TCI Europe) at a 1:1 ratio to produced bead volume. Beads were centrifuged at 1000 relative centrifugal force (rcf) for 1 min, non-aqueous phase was removed, then rinsed with complete growth medium at a 2:1 volume to collected beads and washed again. Any remaining non-aqueous residue was removed by pipette. Final processed beads were stored at 4°C for at least 12 hours in complete growth media to swell to equilibrium size.

In order to measure bead size, an aliquot was taken, diluted 1:10 in complete growth medium, and applied to a glass slide. A transmitted light image was taken using an inverted optical light microscope and 4x objective. Beads were detected in the image using an algorithm based on circular Hough transform. The diameter of the detected beads was then quantified from the image using the established pixel/micron ratio of the calibrated microscope, with a resulting size of 65.8 +/- 6.7 µm (SD).

### 1.3 Production of bead-associated cells

The cells of section 1.1 (figure 1b) were mixed with the hydrogel beads (figure 1a) of section 1.2 minimum 12 hours after processing of beads at a concentration of 10 million cells in complete growth medium / ml and 5.3 million beads / ml. The mixture was then incubated for 2 hours at 37°C to facilitate attachment of the cells to the hydrogel beads (figure 1c) therefor forming bead-associated cells. To prevent sedimentation of cells, the mixture was gently mixed by flicking it with a finger every 30 min.

### 1.4 Formation of ring-shaped tissue scaffold

Ring-shaped tissue scaffolds were formed by casting into agarose molds.

2% agarose (w/v in base DMEM/F12 Glutamax growth medium) was poured into a 6 cm culture dish and a machined mold was used to create annular wells (3 mm inner diameter, 1mm thickness of wells, 3mm depth; figure 3b), a similar approach to others (Strobel, Jove 2018). The agarose molds were sterilized under UV light for 2 hours.

The mixture obtained in section 1.3 (figure 3a) was centrifuged at 150 rcf for 5 min, excess medium was removed and the bead-associated cells were transferred into the agarose molds (figure 3b) by micropipette to a total volume of 30 µl per ring impression, filling the wells to approximately 1 mm height. Rings in the culture dish were cultured in a humidity-controlled incubator at 37°C, 5% CO₂, pO₂ of 141.4 mmHg.

After 30 minutes in the agarose molds, ¹tissue engineering medium (TE medium) was added to the dishes (4 ml in total) gently to avoid disturbing the settled cell-bead mixture. Medium was exchanged at least twice a week. The tissue-engineered rings were maintained for 6 weeks at which time the tissue-engineered rings were self-supporting.

¹Tissue engineering medium (TE medium) consists of the complete growth medium (DMEM/F12 with supplements described in 1.1) with added proline (50 µg/ml) (Paz-Lugo 2018), glycine (50 µg/ml) (Paz-Lugo 2018), alanine (20 µg/ml) (ref), and ascorbic acid (50 µg/ml) (Murad 1981, Berg 1983) to enhance collagen production.

### 1.5 Removal of hydrogel microbeads

Alginate hydrogel beads were removed from the ring constructs obtained in section 1.4 by adding alginate lyase (Sigma, A1603) to the dishes containing the rings to a final concentration of 0.3 mg/ml and incubated for 30 minutes in TE medium. Thereafter, the dish was replenished with fresh TE medium (figure 2d). The tissue-engineered rings were maintained for 6 weeks and medium was exchanged at least twice a week (figure 2e).

### 1.6 Decellularization

Ring constructs obtained in section 1.5 were removed from the agarose mold and placed in a 15 ml conical tube with 1.5% solution of Tween 20 in PBS (10 ml). They were incubated at room temperature in the tube on a rocker (VWR rocker, speed 25, angle 10) for 18 hours. Thereafter, the constructs were transferred to another tube containing 10 ml of 40 U/ml DNase I (Sigma, D4513) diluted in PBS. They were incubated at room temperature in the tube on a rocker (VWR rocker, speed 25, angle 10) for 18 hours. Decellularization was confirmed by imaging after staining with nuclei binding dye, Hoechst. The final product contained no cells as judged by lack of intact nuclei and consists only of cell-derived extracellular matrix.

### Example 2: Self-supporting tissue scaffolds

### 2.1 Tissue culture

Adipose tissue derived mesenchymal stem cells (AT-MSC, Lonza) were cultured in a humidity-controlled incubator at 37°C, 5% CO₂ and supplemented with complete MEM alpha (Gibco Cat. #32561-094) growth medium (supplemented with 10% FBS, 1% Pen-Strep, 10 µg/L hEGF, 720 mg/L hydrocortisone, 17.6 mg/L ascorbic acid, 10 mg/L insulin). Cells were limited to passage less than 10 and sub-cultured prior to full confluency and use in tissue constructs.

### 2.2 Production of hydrogel microbeads

Alginate microbeads were produced using a 2-stream flow focusing droplet chip (Wunderlichips, Switzerland) with a 50x50 µm droplet forming nozzle (figure 1a). The continuous phase consisted of Novec 7500 fluorinated oil (3M, US) containing 1% Pico-Surf (Sphere Fluidics, UK) as a surfactant.

Two dispersed phases were used: (1) 1.0% (wt) total alginate consisting of 0.5% NOVATACH^{™} VLVG 4GRGDSP (4GRGDSP-coupled high G low MW alginate, G/M Ratio ≥ 1.5, nominal viscosity 10-80 mPa.s, 69% guluronate monomer units, 33 kDa) and 0.5% PRONOVA^{™} UP LVG (20-200 mPa.s, 70% guluronate monomer units, 108 kDa), containing 80 mM CaEDTA and 20 mM HEPES at pH 7.0 and (2) 1.0% (wt) total alginate consisting of 0.5% NOVATACH^{™} VLVG 4GRGDSP (4GRGDSP-coupled high G low MW alginate, G/M Ratio ≥ 1.5, nominal viscosity 10-80 mPa.s,69% guluronate monomer units, 33 kDa) and 0.5% PRONOVA^{™} UP LVG (20-200 mPa.s, 70% guluronate monomer units, 108 kDa) containing 80 mM ZnEDDA and 20 mM HEPES at pH 7.0.

The two aqueous phases meet in a co-flow region in the microfluidic channels prior to droplet break-up. The flow rates were set to 1000 µL h⁻¹ for the continuous phase and 250 µL h⁻¹ for both aqueous phases by controlled injection using BD plastic syringes with PTFE plastic tubing (Bohlender) mounted on syringe pumps (New Era, InfusionONE and SyringeTWO).

The microbeads were collected into a tube containing PFO (TCI Europe) (volume equal to 20% of the total continuous phase volume, as calculated from total runtime) and basal growth medium (at 1:1 volume to aqueous phase). Collected beads were centrifuged at 230 relative centrifugal force (rcf) for 2 minutes, the non-aqueous phase was removed, and TE medium is added at a 1:2 volume to collected beads and left to swell. Final processed beads were stored at 4°C for at least 12 hours in the media mixture to swell to equilibrium size.

In order to measure bead size, a 30 ul aliquot was taken, diluted in 70 ul complete growth medium, and applied to a glass slide. A transmitted light image was taken using an inverted optical light microscope and 4x objective. Beads were detected in the image using an algorithm based on circular Hough transform. The diameter of the detected beads was then quantified from the image using the established pixel/micron ratio of the calibrated microscope, with a resulting size of 58.2 +/- 1.7 µm (SD).

Bead concentration is determined by taking a 50 ul aliquot of beads and diluting in 450 ul of TE medium. 10 ul of the bead dilution is transferred to an hemocytometer chamber and manually counted.

### 2.3 Production of bead-associated cells

The cells of section 2.1 (figure 1b) were mixed with the hydrogel beads (figure 1a) of section 2.2 minimum 12 hours after processing of beads, at a concentration of 6.2 million cells / ml in TE medium and 2.9 million beads / ml in TE medium. The mixture was then incubated for 2 hours at 37°C to facilitate attachment of the cells to the hydrogel beads (figure 1c) therefore forming bead-associated cells. To prevent sedimentation of cells, the mixture was gently mixed by flicking it with a finger every 30 min.

### 2.4 Formation of ring-shaped tissue scaffold

Ring-shaped tissue scaffolds were formed by casting into agarose molds.

2% agarose (w/v in base DMEM/F12 Glutamax growth medium) was poured into the wells of a 6-well multi-plate and a machined mold was used to create 2 different sizes of annular wells: 3 mm inner diameter, 1 mm thickness of wells (T1), 3 mm depth; 3 mm inner diameter, 2 mm thickness of wells (T2), 4 mm depth.

The mixture obtained in section 2.3 was centrifuged at 230 rcf for 8 min, excess medium was removed and the bead-associated cells were transferred into the agarose molds by micropipette to a total volume of 30 µl per ring impression for T1 rings and 80 ul for the T2 diameter rings, filling the wells to approximately 1 mm and 2 mm height, respectively. 30 rings in total were filled for each mold size, to allow for 3 rings to be assessed at 10 different timepoints. Rings in the multi-well plate were cultured in a humidity-controlled incubator at 37°C, 5% CO₂, pO₂ of 141.4 mmHg.

After 60 minutes in the agarose molds, TE medium was added to the dishes (4 ml in total) gently to avoid disturbing the settled cell/bead mixture. Medium was exchanged at least twice a week. The tissue-engineered rings were maintained for 5 days to monitor self-supporting properties.

### 2.5 Brightfield imaging and tissue contraction

Brightfield images (Zeiss PrimoVert) were taken of the tissue rings at 1 hour post-molding (immediately after adding medium) (T1: figure 7a; T2: figure 8a), and at 2 hours, 6 hours (T1: figure 7b; T2: figure 8b), 12 hours, 18 hours, 24 hours (T1: figure 7c; T2: figure 8c), 48 hours (T1: figure 7d; T2: figure 8d), 72 hours (T1: figure 7e; T2: figure 8e), 96 hours (T1: figure 7f; T2: figure 8f), and 120 hours post-molding (T1: figure 7g; T2: figure 8g).

Two images were taken of different areas of 3 rings per time point using the 4X objective, ensuring each image contained inner and outer edges of both the formed tissue and the mold. Images were then analyzed taking measurements of the width of the ring and the width of the mold, in order to measure the contraction of the tissue over time (T1: figure 9a; T2: figure 9b).

**Table 1: Contraction of the T1 tissue scaffold over time (figure 9a)**

| **T1** | | | | |
|---|---|---|---|---|
| **Time after molding (hours)** | **Avg/timepoint** | **SD** | -SD | +SD |
| 1 | 1,00201446 | 0,003055926 | 0,998958534 | 1,005070386 |
| 2 | 1,000187458 | 0,002962463 | 0,997224995 | 1,003149922 |
| 6 | 0,878183079 | 0,059698595 | 0,818484484 | 0,937881673 |
| 12 | 0,891641077 | 0,066771101 | 0,824869976 | 0,958412179 |
| 18 | 0,987291743 | 0,01962072 | 0,967671023 | 1,006912463 |
| 24 | 0,882750134 | 0,068080028 | 0,814670107 | 0,950830162 |
| 48 | 0,876822685 | 0,034997231 | 0,841825454 | 0,911819916 |
| 72 | 0,857534138 | 0,022820714 | 0,834713424 | 0,880354852 |
| 96 | 0,687467385 | 0,044608675 | 0,642858711 | 0,73207606 |
| 120 | 0,722655278 | 0,120937287 | 0,601717991 | 0,843592565 |

### Avg/timepoint

*Two images were taken of different areas of 3 rings per time point providing a total of 6 images. Width of the ring and width of the mold were measured for each image. Based on these measurements, the ring (width) : mold(width) ratio was calculated for each image (two images per ring) and average ratio for each ring was calculated. There were 3 rings per timepoint, and the "Avg*/*timepoint value" represents the average ring (width) : mold(width) ratio for these three rings.*

**Table 2: Contraction of the T2 tissue scaffold over time (figure 9b)**

| **T2** | | | | |
|---|---|---|---|---|
| **Time after molding (hours)** | **Avg/timepoint** | **SD** | -SD | +SD |
| 1 | 1,001007325 | 0,003407043 | 0,997600282 | 1,004414368 |
| 2 | 0,995326648 | 0,006170577 | 0,989156071 | 1,001497224 |
| 6 | 0,907257542 | 0,011454595 | 0,895802947 | 0,918712137 |
| 12 | 0,822751441 | 0,017961877 | 0,804789564 | 0,840713319 |
| 18 | 0,836546482 | 0,073009244 | 0,763537238 | 0,909555727 |
| 24 | 0,758118446 | 0,045901323 | 0,712217123 | 0,80401977 |
| 48 | 0,68081303 | 0,036673279 | 0,644139751 | 0,717486309 |
| 72 | 0,717539224 | 0,027262587 | 0,690276637 | 0,744801811 |
| 96 | 0,699961754 | 0,010278051 | 0,689683702 | 0,710239805 |
| 120 | 0,606153645 | 0,035414526 | 0,570739119 | 0,641568171 |

### Avg/timepoint:

*Two images were taken of different areas of 3 rings per time point providing a total of 6 images. Width of the ring and width of the mold were measured for each image. Based on these measurements, the ring (width) : mold(width) ratio was calculated for each image (two images per ring) and average ratio for each ring was calculated. There were 3 rings per timepoint, and the "Avg*/*timepoint value" represents the average ring (width) : mold(width) ratio for these three rings.*

### 2.6 Self-supporting assessment: Manual removal from agarose mold

After imaging, demolding (removal from the molds in the dish) of the 3 rings/time point was attempted using curved forceps. Qualitative results were recorded detailing the rings' self-supporting properties at each time point. The agarose 'wells' containing the rings that were not yet self-supporting were cut-out of the molds and both the contained ring material and excised agarose were transferred together in sample tubes.

While some tissue rings demonstrated some self-supporting properties by 6 hours post-molding, such as floating up from the bottom from the mold along the inner post, they did not withstand handling and removal from the dish using the forceps. By 96 hours post-molding, it was possible to remove the tissue rings from the dish and transfer them while they maintained their ring shape (figure 6) and these rings were therefore considered to be self-supporting within the context of the present application. The average contraction of the T1 tissue rings at this time point is 31.3% and 30% for T2 (68.7 % and 70.0% of their molded width, respectively). Variation and error introduced in these measurements due to movement of some rings in the mold up the inner posts, changing the focal distance and relative width of the ring compared to the edges of the mold area.

## Claims

1. A method of manufacturing a tissue scaffold, the method comprising the following steps:
a) providing a first group of cells and biocompatible hydrogel beads;
b) bringing the first group of cells together with the biocompatible hydrogel beads to obtain a first cell-bead mixture;
c) culturing the first cell-bead mixture to produce extracellular matrix thereby obtaining a first tissue scaffold;
d) optionally, subjecting the first tissue scaffold to mechanical conditioning thereby obtaining a mechanically conditioned first tissue scaffold;
wherein
- the first group of cells are anchorage dependent cells that can produce and secrete extracellular matrix;
- the biocompatible hydrogel beads support attachment of the first group of cells; and
- the first group of cells and the biocompatible hydrogel beads of step b) are brought together in a mold to obtain the first cell-bead mixture; or
the first group of cells and the biocompatible hydrogel beads of step b) are incubated to allow formation of bead-associated cells, the bead-associated cells are then transferred into a mold to obtain the first cell-head mixture

2. The method according to claim 1, wherein the first cell-bead mixture in step c) is cultured at least until the first tissue scaffold is self-supporting.

3. The method according to any one of the preceding claims, wherein step d) is mandatory.

4. The method according to any one of the preceding claims, wherein
- the first tissue scaffold or the mechanically conditioned first tissue scaffold is subjected to means for dissolving or degrading the biocompatible hydrogel beads thereby obtaining a **third tissue scaffold;** or
- the biocompatible hydrogel beads are self-degradable or self-dissolvable; and the first tissue scaffold or the mechanically conditioned first tissue scaffold is cultured until the biocompatible hydrogel beads are self-dissolved or self-degraded, thereby obtaining a **third tissue scaffold.**

5. The method according to claim 4, wherein the third tissue scaffold is subjected to mechanical conditioning thereby obtaining a mechanically conditioned third tissue scaffold.

6. The method according to any one of claims 4 to 5, wherein the third tissue scaffold or the mechanically conditioned third tissue scaffold is subjected to decellularization thereby obtaining a **fourth tissue scaffold.**

7. The method according to claim 6, wherein the fourth tissue scaffold is incubated in the presence of a second group of cells, thereby obtaining a fourth tissue scaffold repopulated with the second group of cells.

8. The method according to any one of the preceding claims, wherein the mold is
i) made of a bioinert material;
ii) designed to allow exchange of nutrients and gases between exterior and interior of the mold; and
iii) completely or partly submerged in cell culture media.

9. A first tissue scaffold comprising
- a first group of cells;
- biocompatible hydrogel beads supporting attachment of the first group of cells; and
- extracellular matrix produced by the first group of cells;
wherein
- the first group of cells are anchorage dependent cells that can produce and secrete extracellular matrix;
- the first group of cells are attached to an outer surface of the biocompatible hydrogel beads; and
- the first group of cells and the biocompatible hydrogel beads are embedded in the extracellular matrix.

10. The method according to any one of claims 1 to 8 or the first tissue scaffold according to claim 9, wherein the biocompatible hydrogel beads are biocompatible alginate beads having one or more cell-adhesion ligand(s) on their outer surface.

11. The method according to any one of claims 1 to 8 or the first tissue scaffold according to any one of claims 9-10, wherein the biocompatible hydrogel beads are biocompatible hydrogel microbeads.

12. The method according to any one of claims 1 to 8 or the first tissue scaffold according to any one of claims 9-11, wherein the biocompatible hydrogel beads are biocompatible hydrogel microbeads of an average size in the range 10 to 200 µm in their largest dimension, more preferably biocompatible hydrogel microbeads of an average size in the range 10 to 150 µm in their largest dimension, even more preferably biocompatible hydrogel microbeads of an average size in the range 30 to 150 µm in their largest dimension and most preferably biocompatible hydrogel microbeads of an average size in the range 30 to 90 µm in their largest dimension.

13. The method according to any one of claims 1 to 8 or the first tissue scaffold according to any one of claims 9-12, wherein at least 60 %, such as at least 70 %, at least 80 % or at least 90 %, of the biocompatible hydrogel beads have a size in the range 10 to 200 µm in their largest dimension, more preferably a size in the range 10 to 150 µm in their largest dimension, even more preferably a size in the range 30 to 150 µm in their largest dimension, and most preferably a size in the range 30 to 90 µm in their largest dimension.

## Patentansprüche

1. Verfahren zum Herstellen eines Gewebegerüsts, wobei das Verfahren die folgenden Schritte umfasst:
a) Bereitstellen einer ersten Gruppe von Zellen und biokompatiblen Hydrogelkügelchen;
b) Zusammenbringen der ersten Gruppe von Zellen mit den biokompatiblen Hydrogelkügelchen, um ein erstes Zellkügelchengemisch zu erhalten;
c) Kultivieren des ersten Zellkügelchengemischs, um extrazelluläre Matrix zu produzieren, wodurch ein erstes Gewebegerüst erhalten wird;
d) optional Aussetzen des ersten Gewebegerüsts mechanischer Konditionierung, wodurch ein mechanisch konditioniertes erstes Gewebegerüst erhalten wird;
wobei
- die erste Gruppe von Zellen verankerungsabhängige Zellen sind, die extrazelluläre Matrix produzieren und sezernieren können;
- die biokompatiblen Hydrogelkügelchen Anbringung der ersten Gruppe von Zellen stützen; und
- die erste Gruppe von Zellen und die biokompatiblen Hydrogelkügelchen aus Schritt b) in einer Form zusammengebracht werden, um das erste Zellkügelchengemisch zu erhalten; oder
die erste Gruppe von Zellen und die biokompatiblen Hydrogelkügelchen aus Schritt b) inkubiert werden, um Bildung von kügelchenassoziierten Zellen zu ermöglichen, wobei die kügelchenassoziierten Zellen dann in eine Form überführt werden, um das erste Zellkügelchengemisch zu erhalten.

2. Verfahren nach Anspruch 1, wobei das erste Zellkügelchengemisch in Schritt c) zumindest kultiviert wird, bis das erste Gewebegerüst selbststützend ist.

3. Verfahren nach einem der vorhergehenden Ansprüche, wobei Schritt d) obligatorisch ist.

4. Verfahren nach einem der vorhergehenden Ansprüche, wobei
- das erste Gewebegerüst oder das mechanisch konditionierte erste Gewebegerüst Mitteln zum Auflösen oder Abbauen der biokompatiblen Hydrogelkügelchen ausgesetzt wird, wodurch ein **drittes Gewebegerüst** erhalten wird; oder
- die biokompatiblen Hydrogelkügelchen selbstabbaubar oder selbstauflösbar sind; und das erste Gewebegerüst oder das mechanisch konditionierte erste Gewebegerüst kultiviert wird, bis die biokompatiblen Hydrogelkügelchen selbstaufgelöst oder selbstabgebaut sind, wodurch ein **drittes Gewebegerüst** erhalten wird.

5. Verfahren nach Anspruch 4, wobei das dritte Gewebegerüst mechanischer Konditionierung ausgesetzt wird, wodurch ein mechanisch konditioniertes drittes Gewebegerüst erhalten wird.

6. Verfahren nach einem der Ansprüche 4 bis 5, wobei das dritte Gewebegerüst oder das mechanisch konditionierte dritte Gewebegerüst Dezellularisierung ausgesetzt wird, wodurch ein **viertes Gewebegerüst** erhalten wird.

7. Verfahren nach Anspruch 6, wobei das vierte Gewebegerüst in der Gegenwart einer zweiten Gruppe von Zellen inkubiert wird, wodurch ein viertes Gewebegerüst erhalten wird, das mit der zweiten Gruppe von Zellen neu bevölkert wird.

8. Verfahren nach einem der vorhergehenden Ansprüche, wobei die Form
i) aus einem bioinerten Material gemacht ist;
ii) gestaltet ist, um Austausch von Nährstoffen und Gasen zwischen Äußerem und Innerem der Form zu ermöglichen; und
iii) ganz oder teilweise in Zellkulturmedien getaucht ist.

9. Erstes Gewebegerüst, umfassend
- eine erste Gruppe von Zellen;
- biokompatible Hydrogelkügelchen, die Anbringung der ersten Gruppe von Zellen stützen; und
- extrazelluläre Matrix, die durch die erste Gruppe von Zellen produziert wird;
wobei
- die erste Gruppe von Zellen verankerungsabhängige Zellen sind, die extrazelluläre Matrix produzieren und sezernieren können;
- die erste Gruppe von Zellen an einer Außenfläche der biokompatiblen Hydrogelkügelchen angebracht ist; und
- die erste Gruppe von Zellen und die biokompatiblen Hydrogelkügelchen in die extrazelluläre Matrix eingebettet sind.

10. Verfahren nach einem der Ansprüche 1 bis 8 oder erstes Gewebegerüst nach Anspruch 9, wobei die biokompatiblen Hydrogelkügelchen biokompatible Alginatkügelchen mit einem oder mehreren Zelladhäsionsligand(en) an ihrer Außenfläche sind.

11. Verfahren nach einem der Ansprüche 1 bis 8 oder erstes Gewebegerüst nach einem der Ansprüche 9-10, wobei die biokompatiblen Hydrogelkügelchen biokompatible Hydrogelmikrokügelchen sind.

12. Verfahren nach einem der Ansprüche 1 bis 8 oder erstes Gewebegerüst nach einem der Ansprüche 9-11, wobei die biokompatiblen Hydrogelkügelchen biokompatible Hydrogelmikrokügelchen mit einer durchschnittlichen Größe in dem Bereich von 10 bis 200 µm in ihrer größten Abmessung, bevorzugter biokompatible Hydrogelmikrokügelchen mit einer durchschnittlichen Größe in dem Bereich von 10 bis 150 µm in ihrer größten Abmessung, noch bevorzugter biokompatible Hydrogelmikrokügelchen mit einer durchschnittlichen Größe in dem Bereich von 30 bis 150 µm in ihrer größten Abmessung und am meisten bevorzugt biokompatible Hydrogelmikrokügelchen mit einer durchschnittlichen Größe in dem Bereich von 30 bis 90 µm in ihrer größten Abmessung sind.

13. Verfahren nach einem der Ansprüche 1 bis 8 oder erstes Gewebegerüst nach einem der Ansprüche 9-12, wobei zumindest 60 %, wie zumindest 70 %, zumindest 80 % oder zumindest 90 %, der biokompatiblen Hydrogelkügelchen eine Größe in dem Bereich von 10 bis 200 µm in ihrer größten Abmessung, bevorzugter eine Größe in dem Bereich von 10 bis 150 µm in ihrer größten Abmessung, noch bevorzugter eine Größe in dem Bereich von 30 bis 150 µm in ihrer größten Abmessung und am meisten bevorzugt eine Größe in dem Bereich von 30 bis 90 µm in ihrer größten Abmessung aufweisen.

## Revendications

1. Procédé de fabrication d'un échafaudage tissulaire, le procédé comprenant les étapes suivantes :
a) la fourniture d'un premier groupe de cellules et de billes d'hydrogel biocompatibles ;
b) la réunion du premier groupe de cellules avec les billes d'hydrogel biocompatibles pour obtenir un premier mélange de cellules et de billes ;
c) la culture du premier mélange de cellules et de billes pour produire une matrice extracellulaire, ce qui permet d'obtenir un premier échafaudage tissulaire ;
d) éventuellement, la soumission du premier échafaudage tissulaire à un conditionnement mécanique, pour obtenir ainsi un premier échafaudage tissulaire conditionné mécaniquement ;
dans lequel
- les cellules du premier groupe sont des cellules dépendantes de l'ancrage qui peuvent produire et sécréter une matrice extracellulaire ;
- les billes d'hydrogel biocompatibles soutiennent la fixation du premier groupe de cellules ; et
- le premier groupe de cellules et les billes d'hydrogel biocompatibles de l'étape b) sont réunis dans un moule pour obtenir le premier mélange de cellules et de billes ; ou
les cellules du premier groupe et les billes d'hydrogel biocompatibles de l'étape b) sont incubées pour permettre la formation de cellules associées aux billes, les cellules associées aux billes sont ensuite transférées dans un moule pour obtenir le premier mélange de cellules et de billes.

2. Procédé selon la revendication 1, ledit premier mélange de cellules et de billes à l'étape c) étant cultivé au moins jusqu'à ce que le premier échafaudage tissulaire soit autoporteur.

3. Procédé selon l'une quelconque des revendications précédentes, dans lequel l'étape d) est obligatoire.

4. Procédé selon l'une quelconque des revendications précédentes, dans lequel
- le premier échafaudage tissulaire ou le premier échafaudage tissulaire conditionné mécaniquement est soumis à des moyens pour dissoudre ou dégrader les billes d'hydrogel biocompatibles, pour obtenir ainsi un troisième échafaudage tissulaire ; ou
- les billes d'hydrogel biocompatibles sont autodégradables ou autodissolubles ; et le premier échafaudage tissulaire ou le premier échafaudage tissulaire conditionné mécaniquement est cultivé jusqu'à ce que les billes d'hydrogel biocompatibles soient autodissoutes ou autodégradées, pour obtenir ainsi un troisième échafaudage tissulaire.

5. Procédé selon la revendication 4, ledit troisième échafaudage tissulaire étant soumis à un conditionnement mécanique, pour obtenir ainsi un troisième échafaudage tissulaire conditionné mécaniquement.

6. Procédé selon l'une quelconque des revendications 4 à 5, dans lequel le troisième échafaudage tissulaire ou le troisième échafaudage tissulaire conditionné mécaniquement est soumis à une décellularisation, pour obtenir ainsi un quatrième échafaudage tissulaire.

7. Procédé selon la revendication 6, dans lequel le quatrième échafaudage tissulaire est incubé en présence d'un second groupe de cellules, pour obtenir ainsi un quatrième échafaudage tissulaire repeuplé avec le second groupe de cellules.

8. Procédé selon l'une quelconque des revendications précédentes, dans lequel le moule est
i) constitué d'un matériau bioinerte ;
ii) conçu pour permettre l'échange de nutriments et de gaz entre l'extérieur et l'intérieur du moule ; et
iii) complètement ou partiellement immergé dans des milieux de culture cellulaire.

9. Premier échafaudage tissulaire comprenant
- un premier groupe de cellules ;
- des billes d'hydrogel biocompatibles soutenant la fixation du premier groupe de cellules ; et
- la matrice extracellulaire produite par le premier groupe de cellules ; dans lequel
- les cellules du premier groupe sont des cellules dépendantes de l'ancrage qui peuvent produire et sécréter une matrice extracellulaire ;
- les cellules du premier groupe sont fixées à une surface extérieure des billes d'hydrogel biocompatibles ; et
- les cellules du premier groupe et les billes d'hydrogel biocompatibles sont noyées dans la matrice extracellulaire.

10. Procédé selon l'une quelconque des revendications 1 à 8 ou premier échafaudage tissulaire selon la revendication 9, dans lequel les billes d'hydrogel biocompatibles sont des billes d'alginate biocompatibles ayant un ou plusieurs ligand(s) d'adhésion cellulaire sur leur surface extérieure.

11. Procédé selon l'une quelconque des revendications 1 à 8 ou premier échafaudage tissulaire selon l'une quelconque des revendications 9 à 10, dans lequel les billes d'hydrogel biocompatibles sont des microbilles d'hydrogel biocompatibles.

12. Procédé selon l'une quelconque des revendications 1 à 8 ou premier échafaudage tissulaire selon l'une quelconque des revendications 9 à 11, dans lequel les billes d'hydrogel biocompatibles sont des microbilles d'hydrogel biocompatibles d'une taille moyenne située dans la plage de 10 à 200 µm dans leur plus grande dimension, plus préférablement des microbilles d'hydrogel biocompatibles d'une taille moyenne située dans la plage de 10 à 150 µm dans leur plus grande dimension, encore plus préférablement des microbilles d'hydrogel biocompatibles d'une taille moyenne située dans la plage de 30 à 150 µm dans leur plus grande dimension et plus préférablement des microbilles d'hydrogel biocompatibles d'une taille moyenne située dans la plage de 30 à 90 µm dans leur plus grande dimension.

13. Procédé selon l'une quelconque des revendications 1 à 8 ou premier échafaudage tissulaire selon l'une quelconque des revendications 9 à 12, dans lequel au moins 60 %, tel qu'au moins 70 %, au moins 80 % ou au moins 90 %, des billes d'hydrogel biocompatibles ont une taille située entre 10 et 200 µm dans leur plus grande dimension, plus préférablement une taille située entre 10 et 150 µm dans leur plus grande dimension, encore plus préférablement une taille située entre 30 et 150 µm dans leur plus grande dimension, et plus préférablement une taille située entre 30 et 90 µm dans leur plus grande dimension.
